# EUROPEAN PATENT SPECIFICATION

(11) **EP 4 058 029 B1**
(45) Date of publication and mention of the grant of the patent: **26.03.2025**
(21) Application number: 20820711.8
(22) Date of filing: 13.11.2020
(51) Int. Cl.: A61K 31/5513, A61K 47/00

(54) **MULTIMODAL COMPOSITIONS AND METHODS OF TREATMENT**
MULTIMODALE ZUSAMMENSETZUNGEN UND VERFAHREN ZUR BEHANDLUNG
COMPOSITIONS MULTIMODALES ET MÉTHODES DE TRAITEMENT

(30) Priority: 14.11.2019 US 201962935460 P
(43) Date of publication of application: 21.09.2022
(73) Proprietor: Aquestive Therapeutics, Inc., Warren, NJ 07059 (US)
(72) Inventor: SCHOBEL, Alexander, Mark, Vero Beach, FL 32963 (US); VARJAN, Stephanie, M., Oradell, NJ 07649 (US); WARGACKI, Stephen, Paul, Pittstown, NJ 08867 (US); SALTKO, Gary, Armonk, NY 10504 (US); HELLER, Allen, H., Woodbridge, CT 06525 (US)
(74) Representative: D Young & Co LLP
(86) International application number: PCT/US2020/060464
(87) International publication number: WO 2021/097247

(56) References cited:
- ROGAWSKI MICHAEL A ET AL: "Diazepam buccal film for the treatment of acute seizures", EPILEPSY AND BEHAVIOR, ACADEMIC PRESS, SAN DIEGO, CA, US, vol. 101, 5 November 2019 (2019-11-05), XP085990428, ISSN: 1525-5050, [retrieved on 20191105], DOI: 10.1016/J.YEBEH.2019.106537
- HEALTH SYNEOS: "A RANDOMIZED, OPEN-LABEL, 2-SEQUENCE, 3-TREATMENT, CROSSOVER STUDY TO EVALUATE THE PHARMACOKINETICS OF SINGLE DOSES OF DIAZEPAM BUCCAL FILM (DBF) (AQUESTIVE THERAPEUTICS) COMPARED WITH DIASTAT RECTAL GEL (VALEANT PHARMACEUTICALS NORTH AMERICA) IN ADULT MALE AND FEMALE SUBJECTS ON A CONCOMITANT REG", 24 May 2019 (2019-05-24), XP055769332, Retrieved from the Internet <URL:https://clinicaltrials.gov/ProvidedDocs/20/NCT03953820/Prot_000.pdf> [retrieved on 20210127]
- STREUBEL A ET AL: "Bimodal drug release achieved with multi-layer matrix tablets: transport mechanisms and device design", JOURNAL OF CONTROLLED RELEASE, ELSEVIER, AMSTERDAM, NL, vol. 69, no. 3, 3 December 2000 (2000-12-03), pages 455 - 468, XP004221295, ISSN: 0168-3659, DOI: 10.1016/S0168-3659(00)00334-5

## Description

### TECHNICAL FIELD

This invention relates to pharmaceutical compositions and methods of treatment.

### BACKGROUND

A seizure is a sudden, uncontrolled electrical disturbance in the brain. It can cause changes in behavior, movements or feelings, and in levels of consciousness. If two or more seizures or a tendency for recurrent seizures occurs, a subject is often diagnosed with epilepsy. Epilepsy is a neurological disorder marked by sudden recurrent episodes of sensory disturbance, loss of consciousness, or convulsions, associated with abnormal electrical activity in the brain. Benzodiazepines are often used to treat medical conditions such as seizures, anxiety, insomnia, alcohol withdrawal, and amnesia. They can be used as a muscle relaxant. They are sometimes provided before an anesthetic, such as before surgery. Some examples of benzodiazepines are alprazolam (Xanax), lorazepam (Ativan), chlordiazepoxide (Librium), and diazepam (Valium). Benzodiazepines can be used to treat CNS (central nervous system) disorders.
Rogawski Michael A et al: "Diazepam buccal film for the treatment of acute seizures" discloses Diazepam buccal film (DBF) for delivering diazepam transbucally and via the gastric route. DBF was found to be a convenient alternative for out-of-hospital treatment of seizure exacerbations. Health Syneos: "A randomized, open-label, 2-sequence, 3- treatment, crossover study to evaluate the pharmacokinetics of single doses of diazepam buccal film (DBF) (aquestive therapeutics) compared with diastat rectal gel (valeant pharmaceuticals north america) in adult male and female subjects on a concomitant reg", is a clinical trial protocol aimed at evaluating the pharmacokinetics of single doses DBF at the recommended dose regimen compared with Diastat^{®} (DRG) at the labeled dose regimen in adult subjects on a stable concomitant regimen of antiepileptic drugs (AED) for the treatment of epilepsy, following a moderate-fat meal.
Streusel A et al: "Bimodal drug release achieved with multi-layer matrix tablets: transport mechanisms and device design", describes new multi-layer matrix tablets to achieve bimodal drug release profiles (fast release/slow release/fast release).

### SUMMARY

The invention is as defined in the appended claims. In particular, the invention relates to diazepam for use in the treatment of seizures, wherein the diazepam is orally administered with a multimodal delivery profile, wherein the diazepam is delivered from a mucoadhesive matrix including a permeation enhancer, wherein permeation of at least a portion of the diazepam through a mucosal tissue is promoted, and wherein a dose of diazepam is adjusted to compensate for a food effect by increasing the dose to offset the food effect such that a subject achieves a safe and efficacious dose in a fed state.

In one embodiment, the multimodal profile is a bimodal delivery profile.

In one embodiment, at least about 5-50 % of the diazepam is administered via an oral transmucosal route.

In one embodiment, at least about 5% of the diazepam is administered via a gastrointestinal route.

In one embodiment, the diazepam is for use in the treatment of generalized seizures, focal seizures, focal aware seizures, focal aware impaired seizures, bilateral tonic seizures, absence seizures, atypical absence seizures, tonic-clonic seizures, atonic seizures, or clonic seizures.

In one embodiment, the diazepam is for use in the treatment of tonic seizures, myoclonic seizures, gelastic and dacrystic seizures, febrile seizures, non-epileptic seizures or refractory seizures.

In one embodiment, the diazepam is administered in a fed state or in a fasted state.

In one embodiment, about 2.5 mg, about 5mg, about 7.5 mg, about 10 mg, about 12.5 mg, about 15 mg, about 17.5 mg, about 20 mg, about 25 mg, or about 30 mg of diazepam is delivered in a single dose.

In one embodiment, a dose of diazepam is administered according to a weight-based regimen. In one embodiment, the matrix is a pharmaceutical film with a residence time of less than 30 minutes, less than 15 minutes, less than 10 minutes or less than 5 minutes in an oral cavity.

Other aspects, embodiments, and features will be apparent from the following description, the drawings, and the claims.

### BRIEF DESCRIPTION OF THE FIGURES

Fig. 1A shows dose-proportional pharmacokinetics of diazepam buccal film (DBF) in healthy adult males in which plasma diazepam concentrations were measured in a single-dose, randomized, open-label, three-period, crossover study of diazepam buccal film (DBF) 5 mg, 10 mg, and 15 mg in 30 healthy adult male volunteers under fasting conditions.
Fig. 1B shows the same study as Fig. 1A for plasma diazepam concentrations measured as a function of time.
Fig. 1C shows a comparison of diazepam buccal film vs. diazepam rectal gel in Cmax as a function of nominal dose.

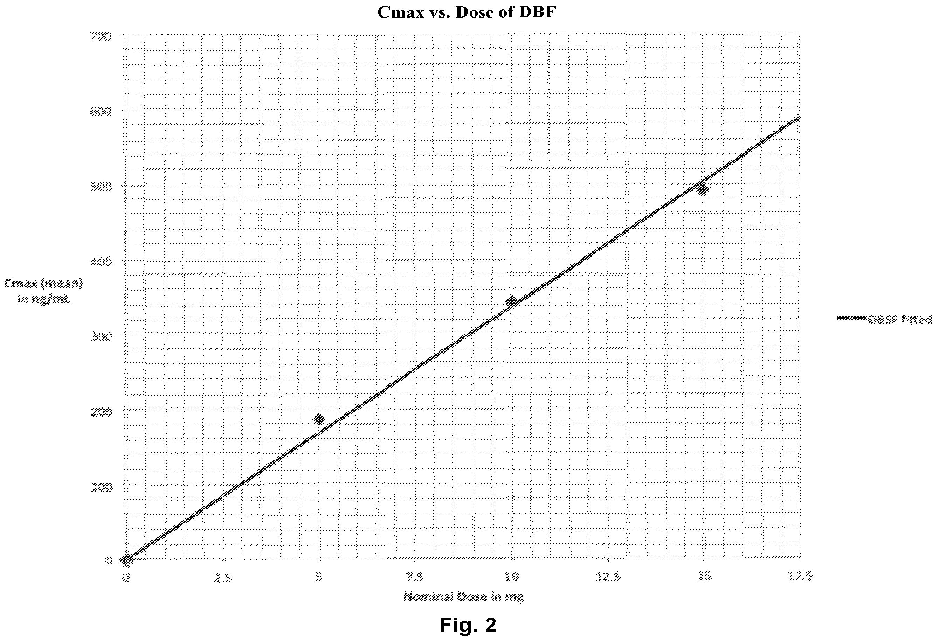

Fig. 2 shows a dose proportionality study that was performed across a 5mg, 10mg and 15mg range for diazepam buccal film.
Fig. 3 shows a pivotal study comparing one DBF dose (15mg) against three doses (5mg, 12.5mg, and 20mg) of the rectal gel.
Fig. 4A shows a study measuring AUC as a function of dose of Diastat^{®}.
Fig. 4B shows a study measuring AUC (dose normalized) as a function of dose of Diastat^{®}.
Fig. 5 shows a food effect study that was performed under fed and fasted conditions.
Fig. 6 shows mean plasma diazepam concentrations in a food effect study comparing fasted upright, fasted reclining, high fat reclining, and moderate fat reclining subjects.
Fig. 7 shows Geometric Mean Diazepam Plasma Concentration Following Administration of DBF and DRG to Adults with Epilepsy According to Body Weight Following a Moderate-Fat Meal (N=28). Geometric mean plasma concentrations from 28 subjects with valid profiles for both DBF and DRG. Error bars are the geometric standard error. Inset shows geometric mean values for Cmax for DBF and DRG with geometric standard deviation.
Fig. 8 shows Cmax (Geometric Mean) by Weight Group (N=28).

### DETAILED DESCRIPTION

Seizure clusters occur in many patients with epilepsy, despite treatment with antiepilepsy medications. Available treatment options remain limited. Benzodiazepines, including diazepam and midazolam, are the mainstay of treatment for seizure emergencies, including acute repetitive seizures. Non-parenteral dosage forms are used when parenteral (intravenous or intramuscular) dosing is not feasible. Currently available non-parenteral dosage forms have limitations in terms of usability, accuracy of dosing, irritation potential, patient and caregiver acceptance, speed of action, and portability. Benzodiazepines can produce toxic effects and can be intentionally or accidentally taken in overdose. Accordingly, a dosing regimen that allows for improved dosing and control, compliance, speed of action, accountability and usability would supply a critical and yet unmet need.

Conventionally, a gel formulation of diazepam intended for rectal administration (e.g., Diastat^{®} Rectal Gel) is administered for certain patients with epilepsy. The drug is administered to patients who require occasional use of diazepam to control bouts of increased seizure activity. Common side effects of this medication include somnolence, sleepiness or drowsiness. Other side effects include dizziness, headache, pain, abdominal pain, nervousness, vasodilation, diarrhea, ataxia or incoordination, euphoria, asthma, rhinitis (irritation of the nose similar to an allergy or a cold) and rash. Diazepam and other currently available products for the treatment of ARS and acute convulsive seizures have significant limitations. Rectal administration is unwieldy, may be embarrassing for patients and caregivers, and use may be restricted by social and legal constraints, and intranasal administration is often poorly accepted by patients due to inaccurate dosing and nasal irritation, which can negatively impact compliance. Most oral tablet forms of benzodiazepines such as lorazepam, diazepam and clonazepam must be swallowed with water and this is only feasible when the patient is awake and alert. Some sublingual or buccal dosage forms may also require patient cooperation depending on the properties of the pharmaceutical composition. Lorazepam in an orodispersible tablet form (Temesta Expidet^{®}) which has been used sublingually in the treatment of acute seizures in children but it and other available oral dosage forms may not act as rapidly as rectal diazepam.

Patients with epilepsy who have worrisome seizure exacerbations outside of a medical facility would benefit from rapid treatment by a caregiver or bystander, or even from a therapy that is self-administered. Such seizure exacerbations fall on a continuum ranging from single breakthrough seizures (including a more severe or prolonged seizure than is typical for the patient), to acute repetitive seizures (ARS), and, in the most severe form, to status epilepticus. ARS, also commonly referred to as seizure clusters or seizure flurries, represents a series of seizures grouped consecutively, typically with short (or shorter than usual) interictal periods. More generally, ARS can be considered a change in frequency of seizures for which treatment is desired. Patients experiencing ARS have drug refractory epilepsy and experience spontaneous seizures on a recurrent basis. When ARS is identified, there is heightened concern for seizure-associated risks including post-ictal psychosis; injury from falls and burns; negative social and pharmacoeconomic impact from frequent emergency department visits, hospitalizations, or missed school or work days; and importantly for status epilepticus that may lead to persistent neurological impairment or death. Parenteral (intravenous or intramuscular) dosing is preferred for seizure rescue therapy, particularly in the emergency treatment of status epilepticus. Alternative non-parental dosage forms are used when parenteral therapy is not feasible or when a patient has such frequent episodes that parenteral therapy is not practical. The objective of therapy may be to prevent seizure recurrence, interrupt progression of a sequence of seizures, or terminate an ongoing seizure.

Oral mucosal drug delivery is an alternative method of systemic drug delivery that offers several advantages over both injectable, nasal, rectal and enteral methods of administration. Because the oral mucosa is highly vascularised, drugs that are absorbed through the oral mucosa directly enter the systemic circulation, bypassing the gastrointestinal tract and first-pass metabolism in the liver. For some drugs, this results in rapid onset of action via a more comfortable and convenient delivery route than the intravenous route. The absorption through the oral mucosa may be 100%. In certain embodiments, the absorption through the oral mucosa may be less than 100%. For example, in certain embodiments, the absorption through the oral mucosa can be approximately less than 90%. It can be approximately less than 80%, less than 70%, less than 60%, less than 50%, less than 40%, less than 30%, less than 20% or less than 10%. In certain embodiments, the absorption through the oral mucosa can be approximately greater than 10%, greater than 20%, greater than 30%, greater than 40%, greater than 50%, greater than 60%, greater than 70%, greater than 80%, or greater than 90%.

Diazepam buccal film (DBF) is a novel formulation that incorporates diazepam in a dissolving polymer matrix in a manner that is compact, portable and designed to be easily administered, with a pharmacokinetic profile comparable to rectally administered diazepam, but with multimodal delivery. Multimodal delivery refers to the fact that the active ingredient is delivered in more than one route, e.g., transmucosal and gastrointestinal routes. DBF can be applied to the buccal mucosa inside the cheek where the film adheres, hydrates and releases diazepam such that it is absorbed transbucally and is also swallowed allowing some enteral absorption as well. Studies showed that bioavailability in adult patients with epilepsy was not significantly different when DBF is applied interictally or periictally (within 5 min of a seizure). DBF is successfully placed and generally used without difficulty, even without patient cooperation immediately after a seizure. In fact, in a crossover comparative study with diazepam rectal gel (Diastat^{®}) in adult patients with epilepsy, DBF performed equivalently to the rectal gel but peak exposures were less variable. DBF is a convenient alternative for out-of-hospital treatment of seizure exacerbations. DBF is also useful to treat acute seizure emergencies. It has been found to be safe and well tolerated by pediatric, adolescent, and adult patients with epilepsy experiencing seizure emergencies, particularly those out of the hospital. DBF was ultimately successfully placed on 99.6% of use occasions and readily used without difficulty when administered by patients and caregivers.

In the present invention, diazepam is administered with a multimodal delivery profile. Diazepam can be administered with a bimodal delivery profile.

The absorption of diazepam through the mucosa is governed by several factors including i) the release rate from the film, ii) the surface area of the film, iii) the residence time of the film and iv) the ability of the molecule to permeate and traverse the oral mucosa to reach the vascular system. Despite significant improvements to the permeation rate, through the incorporation of a penetration enhancer into the formulation, only a portion of the diazepam is delivered transmucosally. The remainder of the diazepam is washed away through salivary flow or swallowed and drains into the gastrointestinal tract where it is absorbed into the body. The combination of these two absorption routes allows DBF to deliver faster diazepam blood levels than can be achieved orally alone but due to the high oral bioavailability, also ensures a complete dose is delivered upon every application. The swallowed portion of the diazepam however is also exposed to variations in the absorption rate due to a well-known food effect.

When delivered orally as in the present invention, diazepam is typically absorbed rapidly and completely by the gastrointestinal tract. Onset of absorption can be as rapid as about 15 min or less and Tmax can also be observed in the range of about 1 hr to 1.5 hours for example. However, after a moderate fat meal, onset of absorption can be delayed to approximately 45 min and Tmax is pushed to about 2-3hours. Along with the delay in absorption comes a reduction in Cmax as absorption is spread out over a longer duration of time. The Cmax is reported to decrease by approximately 28% after a moderate fat meal.

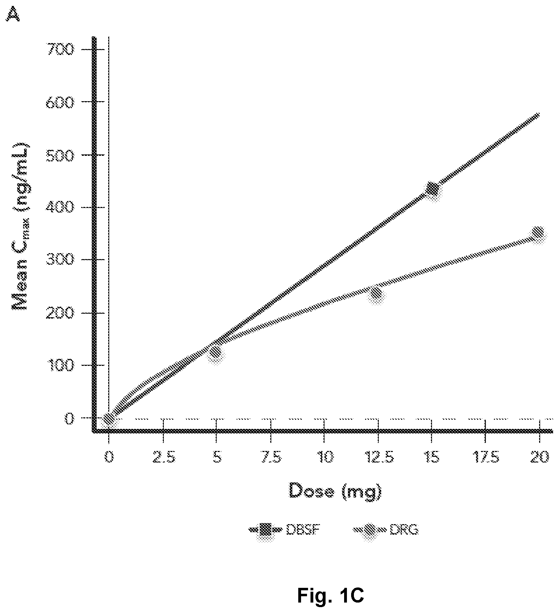

The recommended DBF dose for each weight class as defined in the DRG label was selected (1) to provide a dose sufficiently high to ensure that the predicted median of the resulting diazepam Cmax following a moderate fat meal was similar to the median Cmax following the labeled dose of Diastat rectal gel, and (2) to provide a dose for which the predicted median of the resulting diazepam Cmax under fasting conditions would not exceed the median Cmax values observed and demonstrated as safe in Phase 1 studies with DBF. It was demonstrated that the predicted median diazepam Cmax values with the proposed regimen administered under fasting conditions did not meaningfully exceed the median Cmax values observed in the 104 healthy volunteers (adult men and women) who received DBF 15 mg under fasting conditions in Phase 1 studies conducted by Aquestive. The median Cmax among these 104 healthy subjects (127 DBF administrations) was 467 ng/mL. Under conditions of a moderate fat meal, the proposed DBF dosing regimen produces a Cmax similar to the Cmax expected following the corresponding labeled dose of Diastat rectal gel (DRG). Ultimately, high and low dose formulations were tested in pilot clinical trials against DRG at 5mg and 20mg strengths. The results showed excellent agreement between the 5mg DBF and 5mg DRG and also supported dose proportionality between the 20mg DBF and 5mg DBF. This is in contrast to the lack of proportionality between the 5mg DRG to the 20mg DRG as shown in Fig. 1C. The20 mg DBF was predicated to achieve about 367 ng/ml, but unexpectedly achieved over 600 ng/ml, or over 180% of the Target Cmax.

Unexpectedly, DBF can reach a therapeutic window within 1 hour or less. In certain conditions, DBF can reach a therapeutic window within 45 minutes, within 30 minutes, within 15 minutes, within 10 minutes, or within 5 minutes or less. In certain conditions, DBF can reach a therapeutic window in 5 minutes or more. In certain conditions, DBF can reach a therapeutic window in greater than 10 minutes, greater than 15 minutes, greater than 30 minutes, or greater than 45 minutes. In a therapeutic window for DBF, blood levels of no less than 100 ng/ml, 90 ng/ml, 80 ng/ml, 70 ng/ml, 60 ng/ml, 50 ng/ml, 40 ng/ml, 30 ng/ml. 20 ng/ml, or 10 ng/ml DBF can be observed. In certain embodiments, a therapeutic window for DBF results in blood levels of no more than 10 ng/ml, 20 ng/ml, 30 ng/ml, 40 ng/ml, 50 ng/ml, 60 ng/ml, 70 ng/ml, 80 ng/ml. 90 ng/ml, or 100 ng/ml DBF can be observed.

In addition, 17.5 mg DBF unexpectedly delivers same bioavailability as DRG. Moreover, 17.5 mg DBF was also found unexpectedly deliver the same Cmax under moderate fat conditions, as 20 mg DRG.

DBF differed from DRG in the following respects (1) DBF exhibited higher bioavailability than DRG; (2) The PK behavior of DBF was linear. Specifically, for DBF both Cmax and AUC increased in proportion to the dose. In contrast, the PK behavior of DRG was not linear. Specifically, for DRG, Cmax increased with dose to a degree that was less than dose-proportional, whereas AUC increased in proportion to the dose. (3) DBF exhibited a food effect (~45% reduction on average in Cmax after a high fat meal and ~33% reduction on average after a moderate fat meal with no change in AUC). In contrast, it is assumed that DRG, because of its rectal route of administration, is not affected by food. There can be less intersubject variability with a transmucosal or transbuccal film as compared to a rectally delivered Diastat gel.

Applicants used population PK modeling to select a dosing regimen to compensate for the differences in PK between DBF and DRG as shown in the chart below.

| **Weight (Kg)** | **DRG (mg)** | **DBF Weight - Adjusted (mg)** |
|---|---|---|
| 38 to 50 | 10 | 10 |
| 51 to 62 | 12.5 | 12.5 |
| 63 to 75 | 15 | 15 |
| 76 to 87 | 17.5 | 15 |
| 88 to 111 | 20 | 17.5 |

In brief, the recommended DBF dose corresponding to each weight class as defined in the Diastat rectal gel label was selected (1) to provide a dose sufficiently high to ensure that the predicted median of the resulting diazepam Cmax following a moderate fat meal was similar to the median Cmax following the labeled dose of Diastat rectal gel, and (2) to provide a dose for which the predicted median of the resulting diazepam Cmax under fasting conditions would not exceed the median Cmax values observed and demonstrated as safe in Phase 1 healthy volunteer studies with DBF. Simulations based on population PK modeling demonstrated that under conditions of a moderate fat meal, the proposed DBF dosing regimen produced for each weight class a Cmax similar to the Cmax expected following the labeled dose of Diastat rectal gel.

In another example, the dosing regime can be as indicated in the following charts:

| **Weight (Kg)** | **DRG (mg)** | **DBF Weight - Adjusted (mg)** |
|---|---|---|
| 14 to 25 | 5 | 5 |
| 26 to 38 | 7.5 | 7.5 |
| 38 to 50 | 10 | 10 |
| 51 to 62 | 12.5 | 12.5 |
| 63 to 75 | 15 | 15 |
| 76 to 87 | 17.5 | 15 |
| 88 to 111 | 20 | 17.5 |

| **Weight (Kg)** | **DRG (mg)** | **DBF Weight - Adjusted (mg)** |
|---|---|---|
| 14 to 25 | 5 | 5 |
| 26 to 38 | 7.5 | 7.5 |
| 38 to 50 | 10 | 10 |
| 51 to 62 | 12.5 | 12.5 |
| 63 to 75 | 15 | 15 |
| 76 to 87 | 17.5 | 17.5 |
| 88 to 111 | 20 | 17.5 |

| **Weight (Kg)** | **DRG (mg)** | **DBF Weight - Adjusted (mg)** |
|---|---|---|
| 14 to 25 | 5 | 5 |
| 26 to 38 | 7.5 | 7.5 |
| 38 to 50 | 10 | 10 |
| 51 to 62 | 12.5 | 15 |
| 63 to 75 | 15 | 15 |
| 76 to 87 | 17.5 | 17.5 |
| 88 to 111 | 20 | 17.5 |

| **Weight (Kg)** | **DRG (mg)** | **DBF Weight - Adjusted (mg)** |
|---|---|---|
| 14 to 25 | 5 | 5 |
| 26 to 38 | 7.5 | 10 |
| 38 to 50 | 10 | 10 |
| 51 to 62 | 12.5 | 15 |
| 63 to 75 | 15 | 15 |
| 76 to 87 | 17.5 | 17.5 |
| 88 to 111 | 20 | 17.5 |

| **Weight (Kg)** | **DRG (mg)** | **DBF Weight - Adjusted (mg)** |
|---|---|---|
| 14 to 25 | 5 | 5 |
| 26 to 38 | 7.5 | 10 |
| 38 to 50 | 10 | 12.5 |
| 51 to 62 | 12.5 | 15 |
| 63 to 75 | 15 | 15 |
| 76 to 87 | 17.5 | 17.5 |
| 88 to 111 | 20 | 17.5 |

| **Weight (Kg)** | **DRG (mg)** | **DBF Weight - Adjusted (mg)** |
|---|---|---|
| 14 to 25 | 5 | 5 |
| 26 to 38 | 7.5 | 10 |
| 38 to 50 | 10 | 12.5 |
| 51 to 62 | 12.5 | 15 |
| 63 to 75 | 15 | 17.5 |
| 76 to 87 | 17.5 | 17.5 |
| 88 to 111 | 20 | 20 |

| **Weight (Kg)** | **DRG (mg)** | **DBF Weight - Adjusted (mg)** |
|---|---|---|
| 14 to 25 | 5 | 7.5 |
| 26 to 38 | 7.5 | 10 |
| 38 to 50 | 10 | 12.5 |
| 51 to 62 | 12.5 | 15 |
| 63 to 75 | 15 | 17.5 |
| 76 to 87 | 17.5 | 17.5 |
| 88 to 111 | 20 | 20 |

Population pharmacokinetic modeling was used to model the pharmacokinetic profiles for DBF and Diastat rectal gel under fasted and fed conditions. The modeling can show acceptable profiles under fasting conditions, after a moderate fat meal, or after a high fat meal. The profiles can differ for male subjects and female subjects. Female subjects can have lower plasma concentrations. The method of treating a medical condition includes delivering 0.1-0.4 mg/kg of diazepam, for example, 0.1-0.3 mg/kg of diazepam.

In some embodiments, the dose can be lower than the comparable Diastat dose for a weight class of subject, for example, in the fasted state. In other embodiments, the dose can be higher than the comparable Diastat dose for a weight class of subject, for example, in the fed state.

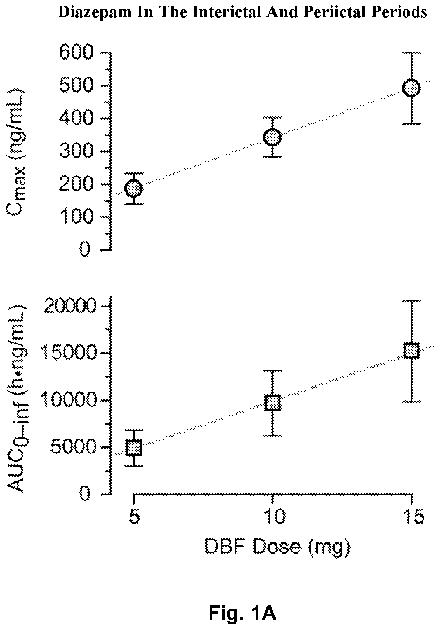

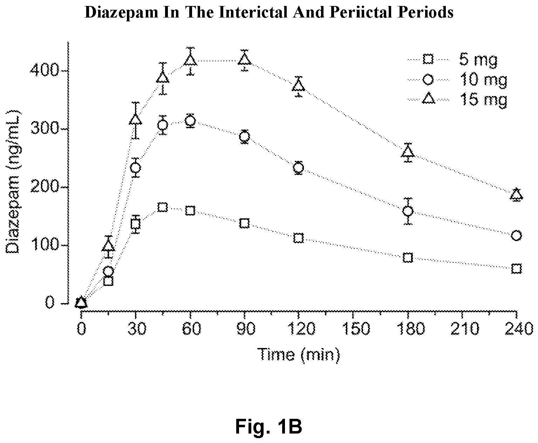

Referring to **Figure 1A** and **Figure 1B** (same study), plasma diazepam concentrations were measured in a single-dose, randomized, open-label, three-period, crossover study of diazepam buccal film (DBF) 5 mg, 10 mg, and 15 mg in 30 healthy adult male volunteers under fasting conditions. Data points represent mean ± S.D. maximum plasma concentration (Cmax) and extrapolated area under the concentration-time curve from time zero to infinity (AUC0-inf). Best fit straight lines show that both values increase linearly with dose.

Referring to **Figure 1A****,** DBF doses of 5 mg to 15 mg exhibited rapid absorption and linear dose-proportional pharmacokinetics. By contrast, diazepam rectal gel showed sublinear dose-proportional pharmacokinetics for Cmax. Recent studies in animal models indicate that plasma diazepam concentrations in the range of 70 ng/ml are associated with an elevation in seizure threshold. *See, e.g.* Dhir A, Rogawski MA. Determination of minimal steady-state plasma level of diazepam causing seizure threshold elevation in rats. Epilepsia. 2018 May; 59(5):935-944. doi: 10.1111/epi.14069. Epub 2018 Apr 6. PubMed PMID: 29682729; PubMed Central PMCID: PMC5934328. At 15 min after application to the buccal mucosa, the mean plasma concentration following a 15 mg DBF dose exceeds this level (70 ng/ml).

Referring to **Figure 1B****,** Applicants found that there was no significant difference in the exposures to diazepam in the interictal and periictal periods, demonstrating adequate performance of DBF even when administered to the buccal mucosa in the period immediately after a seizure. Thirty healthy adult males ages 18-55, body weight 83.5 ± 11.4 kg and BMI 26.7 ± 2.2 kg/m2 (mean ± S.D.), were administered single DBF doses containing 5 mg, 10 mg or 15 mg diazepam under fasting conditions in an open-label, 3-period, randomized sequence crossover study with 21 day washout between treatments. Referring to Figure 1A, each subject received at least one DBF dose. Data points represent the mean ± S.E.M. of 25 to 30 plasma concentration measurements during the 4-h period after dosing. Error bars are not shown when they are smaller than the size of the symbols.

The straight-line interpolated Cmax value for a 12.5 mg DBF dose in fasted healthy volunteers based on the data presented in Fig. 1A is 417 ng/mL, which is substantially greater than the geometric mean Cmax values obtained in the study with subjects with epilepsy. Diazepam is N-demethylated by CYP3A4 and CYP2C19 so that its clearance is increased by inducers of these isozymes. *See, e.g.,* Riss J, Cloyd J, Gates J, Collins S. Benzodiazepines in epilepsy: pharmacology and pharmacokinetics. Acta Neurol Scand. 2008 Aug;118(2):69-86. doi: 10.1111/j.1600-0404.2008.01004.x. Epub 2008 Mar 31. Review. PubMed PMID: 18384456. The lower than expected Cmax value obtained in subjects with epilepsy could be due in part to concomitant inducing antiseizure drugs taken by many of the subjects. There was also a reduced AUC0-inf consistent with CYP induction. Additional as yet undefined factors could also play a role in the reduced mean Cmax value in subjects with epilepsy, including an effect of food on the rate of drug absorption since subjects were not fasted.

The food effect observed after administration of DBF is more pronounced than that described for orally administered diazepam. It is postulated that this is a result of the portion of the dose being absorbed through the buccal mucosa and a portion being absorbed orally. This portion of the dose is not subject to a food effect and therefore, in the fed state only a portion of the absorption profile is moved to longer times. The Cmax of the resulting profile which, under fasting conditions is a combination of both routes of absorption, is unexpectedly reduced because the portion orally absorbed is not only reduced due to the presence of the food, but the profile is decoupled from buccal absorption profile.

### Permeation Enhancers

Solubility and permeability of the pharmaceutically active component in vivo, in particular, in the mouth of a subject, can vary tremendously. A particular class of permeation enhancer can improve the uptake and bioavailability of the pharmaceutically active component in vivo. In particular, when delivered to the mouth via a film, the permeation enhancer can improve the permeability of the pharmaceutically active component through the mucosa and into the blood stream of the subject. The permeation enhancer can improve absorption rate and amount of the pharmaceutically active component by more than 5%, more than 10%, more than 20%, more than 30%, more than 40%, more than 50%, more than 60%, more than 70%, more than 80%, more than 90%, more than 100%, more than 150%, about 200% or more, or less than 200%, less than 150%, less than 100%, less than 90%, less than 80%, less than 70%, less than 60%, less than 50%, less than 40%, less than 30%, less than 20%, less than 10%, or less than 5%, or a combination of these ranges, depending on the other components in the composition. Examples of suitable permeation enhancers are shown, for example, U.S. Patent Application 15/587,364 and U.S. Patent Application 15/791,249.

In certain embodiments, a pharmaceutical composition has a suitable nontoxic, nonionic alkyl glycoside having a hydrophobic alkyl group joined by a linkage to a hydrophilic saccharide in combination with a mucosal delivery-enhancing agent selected from: (a) an aggregation inhibitory agent; (b) a charge-modifying agent; (c) a pH control agent; (d) a degradative enzyme inhibitory agent; (e) a mucolytic or mucus clearing agent; (f) a ciliostatic agent; (g) a membrane penetration-enhancing agent selected from: (i) a surfactant; (ii) a bile salt; (ii) a phospholipid additive, mixed micelle, liposome, or carrier; (iii) an alcohol; (iv) an enamine; (v) an NO donor compound; (vi) a long chain amphipathic molecule; (vii) a small hydrophobic penetration enhancer; (viii) sodium or a salicylic acid derivative; (ix) a glycerol ester of acetoacetic acid; (x) a cyclodextrin or beta-cyclodextrin derivative; (xi) a medium-chain fatty acid; (xii) a chelating agent; (xiii) an amino acid or salt thereof; (xiv) an N-acetylamino acid or salt thereof; (xv) an enzyme degradative to a selected membrane component; (ix) an inhibitor of fatty acid synthesis; (x) an inhibitor of cholesterol synthesis; and (xi) any combination of the membrane penetration enhancing agents recited in (i)-(x); (h) a modulatory agent of epithelial junction physiology; (i) a vasodilator agent; (j) a selective transport-enhancing agent; and (k) a stabilizing delivery vehicle, carrier, mucoadhesive, support or complex-forming species with which the compound is effectively combined, associated, contained, encapsulated or bound resulting in stabilization of the compound for enhanced transmucosal delivery, wherein the formulation of the compound with the transmucosal delivery-enhancing agents provides for increased bioavailability of the compound in blood plasma of a subject. Penetration enhancers have been described in J. Nicolazzo, et al., J. of Controlled Disease, 105 (2005) 1-15.

### Oral Mucosa

There are many reasons why the oral mucosa might be an attractive site for the delivery of therapeutic agents into the systemic circulation. Due to the direct drainage of blood from the buccal epithelium into the internal jugular vein first-pass metabolism in the liver and intestine may be avoided. First-pass effect can be a major reason for the poor bioavailability of some compounds when administered orally. Additionally, the mucosa lining in the oral cavity is easily accessible, which ensures that a dosage form can be applied to the required site and can be removed easily in the case of an emergency. However, like the skin, the buccal mucosa acts as a barrier to the absorption of xenobiotics, which can hinder the permeation of compounds across this tissue. The presence of no or little oral irritation is also a factor that is important when utilizing a transmucosal delivery system. Consequently, the identification of safe and effective penetration enhancers has become a major goal in the quest to improve oral mucosal drug delivery.

Chemical penetration enhancers are substances that control the permeation rate of a coadministered or sequentially administered drug through a biological membrane. While extensive research has focused on obtaining an improved understanding of how penetration enhancers might alter intestinal and transdermal permeability, far less is known about the mechanisms involved in buccal and sublingual penetration enhancement.

The buccal mucosa delineates the inside lining of the cheek as well as the area between the gums and upper and lower lips and it has an average surface area of 100 cm². The surface of the buccal mucosa consists of a stratified squamous epithelium which is separated from the underlying connective tissue (lamina propria and submucosa) by an undulating basement membrane (a continuous layer of extracellular material approximately 1-2 µm in thickness). This stratified squamous epithelium consists of differentiating layers of cells which change in size, shape, and content as they travel from the basal region to the superficial region, where the cells are shed. There are approximately 40-50 cell layers, resulting in a buccal mucosa which is 500-600 µm thick.

Structurally the sublingual mucosa is comparable to the buccal mucosa but the thickness of this epithelium is 100-200 µm. This membrane is also non-keratinised and being relatively thinner has been demonstrated to be more permeable than buccal mucosa. Blood flow to the sublingual mucosal is slower compared with the buccal mucosa and is of the order of 1.0 ml/ min⁻¹/cm⁻². However, the salivary flow to the sublingual region is greater than the buccal mucosa thereby offering challenges that can prematurely cause dilution and subsequent swallowing of the drug before it can penetrate the sublingual mucosa.

The permeability of the buccal mucosa is greater than that of the skin, but less than that of the intestine. The differences in permeability are the result of structural differences between each of the tissues. The absence of organized lipid lamellae in the intercellular spaces of the buccal mucosa results in greater permeability of exogenous compounds, compared to keratinized epithelia of the skin; while the increased thickness and lack of tight junctions results in the buccal mucosa being less permeable than intestinal tissue.

The primary barrier properties of the buccal mucosa have been attributed to the upper one-third to one-quarter of the buccal epithelium. Researchers have learned that beyond the surface epithelium, the permeability barrier of nonkeratinized oral mucosa could also be attributed to contents extruded from the membrane-coating granules into the epithelial intercellular spaces.

The intercellular lipids of the nonkeratinized regions of the oral cavity are of a more polar nature than the lipids of the epidermis, palate, and gingiva, and this difference in the chemical nature of the lipids may contribute to the differences in permeability observed between these tissues. Consequently, it appears that it is not only the greater degree of intercellular lipid packing in the stratum corneum of keratinized epithelia that creates a more effective barrier, but also the chemical nature of the lipids present within that barrier.

### Paracellular and Transcellular Transport

The existence of hydrophilic and lipophilic regions in the oral mucosa has led researchers to postulate the existence of two routes of drug transport through the buccal mucosa which are paracellular (between the cells) and transcellular (across the cells).

Since drug delivery through the buccal mucosa is limited by the barrier nature of the epithelium and the area available for absorption, various enhancement strategies are required in order to deliver therapeutically relevant amounts of drug to the systemic circulation. Various methods, including the use of chemical penetration enhancers, more permeable prodrugs, and physical methods, may be employed to overcome the barrier properties of the buccal mucosa.

A chemical penetration enhancer, or absorption promoter, is a substance added to a pharmaceutical formulation in order to increase the membrane permeation or absorption rate of the coadministered drug, without damaging the membrane and/or causing toxicity. There have been many studies investigating the effect of chemical penetration enhancers on the delivery of compounds across the skin, nasal mucosa, and intestine. In recent years, more attention has been given to the effect of these agents on the permeability of the buccal mucosa. Since permeability across the buccal mucosa is considered to be a passive diffusion process the steady state flux (Jss) should increase with increasing donor chamber concentration (CD) according to Fick's first law of diffusion.

### Surfactants, Bile Salts and Other Permeation Enhancers

Surfactants and bile salts have been shown to enhance the permeability of various compounds across the oral mucosa, both in vitro and in vivo. Aromatic and aliphatic alcohols, for example, benzyl alcohol, can enhance the permeability of various compounds across the oral mucosa, both in vitro and in vivo. The data obtained from these studies strongly suggest that the enhancement in permeability is due to an effect of the surfactants on the mucosal intercellular lipids. The permeation enhancers can be applied in an oral mucosa, e.g. in buccal or sublingual administration. A permeation enhancer can be a synthetic compound. In certain embodiments, a permeation enhancer can be a biosynthetic compound. In certain embodiments, a permeation enhancer can be a natural compound. In other embodiments, a permeation enhancer can include a combination of compounds from one or more of these categories of compounds.

Fatty acids have been shown to enhance the permeation of a number of drugs through the skin, and this has been shown by differential scanning calorimetry and Fourier transform infrared spectroscopy to be related to an increase in the fluidity of intercellular lipids.

Additionally, pretreatment with ethanol has been shown to enhance the permeability of tritiated water and albumin across ventral tongue mucosa, and to enhance caffeine permeability across porcine buccal mucosa. There are also several reports of the enhancing effect of Azone^{®} on the permeability of compounds through oral mucosa. Further, chitosan, a biocompatible and biodegradable polymer, has been shown to enhance drug delivery through various tissues, including the intestine and nasal mucosa.

Oral transmucosal drug delivery (OTDD) is the administration of pharmaceutically active agents through the oral mucosa to achieve systemic effects. Permeation pathways and predictive models for OTDD are described, e.g. in M. Sattar, Oral transmucosal drug delivery - Current status and future prospects, Int'l. Journal of Pharmaceutics, 47(2014) 498-506. OTDD continues to attract the attention of academic and industrial scientists. Despite limited characterization of the permeation pathways in the oral cavity compared with skin and nasal routes of delivery, recent advances in our understanding of the extent to which ionized molecules permeate the buccal epithelium, as well as the emergence of new analytical techniques to study the oral cavity, and the progressing development of in silico models predictive of buccal and sublingual permeation, prospects are encouraging.

In order to deliver broader classes of drugs across the buccal mucosa, reversible methods of reducing the barrier potential of this tissue should be employed. This requisite has fostered the study of penetration enhancers that will safely alter the permeability restrictions of the buccal mucosa. It has been shown that buccal penetration can be improved by using various classes of transmucosal and transdermal penetration enhancers such as bile salts, surfactants, fatty acids and their derivatives, chelators, cyclodextrins and chitosan. Among these chemicals used for the drug permeation enhancement, bile salts are the most common.

In vitro studies on enhancing effect of bile salts on the buccal permeation of compounds is discussed in Sevda Senel, Drug permeation enhancement via buccal route: possibilities and limitations, Journal of Controlled Release 72 (2001) 133-144. That article also discusses recent studies on the effects of buccal epithelial permeability of dihydroxy bile salts, sodium glycodeoxycholate (SGDC) and sodium taurodeoxycholate (TDC) and tri-hydroxy bile salts, sodium glycocholate (GC) and sodium taurocholate (TC) at 100 mM concentration including permeability changes correlated with the histological effects. Fluorescein isothiocyanate (FITC), morphine sulfate were each used as the model compound. Chitosan has also been shown to promote absorption of small polar molecules and peptide / protein drugs through nasal mucosa in animal models and human volunteers. Other studies have shown an enhancing effect on penetration of compounds across the intestinal mucosa and cultured Caco-2 cells.

The permeation enhancer can be a phytoextract. A phytoextract can be an essential oil or composition including essential oils extracted by distillation of the plant material. In certain circumstances, the phytoextract can include synthetic analogues of the compounds extracted from the plant material (i.e., compounds made by organic synthesis). The phytoextract can include a phenylpropanoid, for example, phenyl alanine, eugenol, eugenol acetate, a cinnamic acid, a cinnamic acid ester, a cinnamic aldehyde, a hydrocinnamic acid, chavicol, or safrole, or a combination thereof. The phytoextract can be an essential oil extract of a clove plant, for example, from the leaf, stem or flower bud of a clove plant. The clove plant can be *Syzygium aromaticum.* The phytoextract can include 20-95% eugenol, including 40-95% eugenol, including 60-95% eugenol, and for example, 80-95% eugenol. The extract can also include 5% to 15% eugenol acetate. The extract can also include caryophyllene. The extract can also include up to 2.1% α-humulen. Other volatile compounds included in lower concentrations in clove essential oil can be β-pinene, limonene, farnesol, benzaldehyde, 2-heptanone and ethyl hexanoate. Other permeation enhancers may be added to the composition to improve absorption of the drug. Suitable permeation enhancers include natural or synthetic bile salts such as sodium fusidate; glycocholate or deoxycholate and their salts; fatty acids and derivatives such as sodium laurate, oleic acid, oleyl alcohol, monoolein, and palmitoylcarnitine; chelators such as disodium EDTA, sodium citrate and sodium lauryl sulfate, azone, sodium cholate, sodium 5-methoxysalicylate, sorbitan laurate, glyceryl monolaurate, octoxynonyl-9, laureth-9, polysorbates, sterols, or glycerides, such as caprylocaproyl polyoxylglycerides, e.g., Labrasol. The permeation enhancer can include phytoextract derivatives and/or monolignols. The permeation enhancer can also be a fungal extract.

Some natural products of plant origin have been known to have a vasodilatory effect. There are several mechanisms or modes by which plant-based products can evoke vasodilation. For review, see McNeill J.R. and Jurgens, T.M., Can. J. Physiol. Pharmacol. 84:803-821 (2006. Specifically, vasorelaxant effects of eugenol have been reported in a number of animal studies. See, e.g., Lahlou, S., et al., J. Cardiovasc. Pharmacol. 43:250-57 (2004), Damiani, C.E.N., et al., Vascular Pharmacol. 40:59-66 (2003), Nishijima, H., et al., Japanese J. Pharmacol. 79:327-334 (1998), and Hume W.R., J. Dent Res. 62(9):1013-15 (1983). Calcium channel blockade was suggested to be responsible for vascular relaxation induced by a plant essential oil, or its main constituent, eugenol. See, Interaminense L.R.L. et al., Fundamental & Clin. Pharmacol. 21: 497-506 (2007).

Fatty acids can be used as inactive ingredients in drug preparations or drug vehicles. Fatty acids can also be used as formulation ingredients due to their certain functional effects and their biocompatible nature. Fatty acid, both free and as part of complex lipids, are major metabolic fuel (storage and transport energy), essential components of all membranes and gene regulators. For review, see Rustan A.C. and Drevon, C.A., Fatty Acids: Structures and Properties, Encyclopedia of Life Sciences (2005). There are two families of essential fatty acids that are metabolized in the human body: ω-3 and ω-6 polyunsaturated fatty acids (PUFAs). If the first double bond is found between the third and the fourth carbon atom from the ω carbon, they are called ω-3 fatty acids. If the first double bond is between the sixth and seventh carbon atom, they are called ω-6 fatty acids. PUFAs are further metabolized in the body by the addition of carbon atoms and by desaturation (extraction of hydrogen). Linoleic acid, which is a ω-6 fatty acid, is metabolized to γ-linolenic acid, dihomo-γ-linolinic acid, arachidonic acid, adrenic acid, tetracosatetraenoic acid, tetracosapentaenoic acid, docosapentaenoic acid, α-linolenic acid, which is a ω-3 fatty acid is metabolized to octadecatetraenoic acid, eicosatetraenoic acid, eicosapentaenoic acid (EPA), docosapentaenoic acid, tetracosapentaenoic acid, tetracosahexaenoic acid and docosahexaenoic acid (DHA).

It has been reported that fatty acids, such as palmitic acid, oleic acid, linoleic acid and eicosapentaenoic acid, induced relaxation and hyperpolarization of porcine coronary artery smooth muscle cells via a mechanism involving activation of the Na⁺K⁺-APTase pump and the fatty acids with increasing degrees of cis-unsaturation had higher potencies. See, Pomposiello, S.I. et al., Hypertension 31:615-20 (1998). Interestingly, the pulmonary vascular response to arachidonic acid, a metabolite of linoleic acid, can be either vasoconstrictive or vasodilative, depending on the dose, animal species, the mode of arachidonic acid administration, and the tones of the pulmonary circulation. For example, arachidonic acid has been reported to cause cyclooxygenase-dependent and -independent pulmonary vasodilation. See, Feddersen, C.O. et al., J. Appl. Physiol. 68(5):1799-808 (1990); and see, Spannhake, E.W., et al., J. Appl. Physiol. 44:397-495 (1978) and Wicks, T.C. et al., Circ. Res. 38:167-71 (1976).

Many studies have reported effects of EPA and DHA on vascular reactivity after being administered as ingestible forms. Some studies found that EPA-DHA or EPA alone suppressed the vasoconstrictive effect of norepinephrine or increased vasodilatory responses to acetylcholine in the forearm microcirculation. See, Chin, J.P.F, et al., Hypertension 21:22-8 (1993), and Tagawa, H. et al., J Cardiovasc Pharmacol 33:633-40 (1999). Another study found that both EPA and DHA increased systemic arterial compliance and tended to reduce pulse pressure and total vascular resistance. See, Nestel, P. et al., Am J. Clin. Nutr. 76:326-30 (2002). Meanwhile, a study found that DHA, but not EPA, enhanced vasodilator mechanisms and attenuates constrictor responses in forearm microcirculation in hyperlipidemic overweight men. See, Mori, T.A., et al., Circulation 102:1264-69 (2000). Another study found vasodilator effects of DHA on the rhythmic contractions of isolated human coronary arteries *in vitro.* See Wu, K.-T. et al., Chinese J. Physiol. 50(4):164-70 (2007).

### Sequence of Permeation Enhancer(s) and Active Pharmaceutical Ingredient(s)

The arrangement, order, or sequence of penetration enhancer(s) and active pharmaceutical ingredient(s) (API(s) - diazepam in the present invention) delivered to the desired mucosal surface can vary in order to deliver a desired pharmacokinetic profile. For example, one can apply the permeation enhancer(s) first by a film, by swab, spray, gel, rinse or by a first layer of a film then apply the API(s) by single film, by swab, or by a second layer of a film. The sequence can be reversed or modified, for example, by applying the API(s) first by film, by swab, or by a first layer of a film, and then applying the permeation enhancer(s) by a film, by swab, spray, gel, rinse or by a second layer of a film. In another embodiment, one may apply a permeation enhancer(s) by a film, and a drug by a different film. For example, the permeation enhancer(s) film positioned under a film containing the API(s), or the film containing the API(s) positioned under a film containing the permeation enhancer(s), depending on the desired pharmacokinetic profile.

For example, the penetration enhancer(s) can be used as a pretreatment alone or in combination with at least one API to precondition the mucosa for further absorption of the API(s). The treatment can be followed by another treatment with neat penetration enhancer(s) to follow the at least one API mucosal application. The pretreatment can be applied as a separate treatment (film, gel, solution, swab etc.) or as a layer within a multilayered film construction of one or more layers. Similarly, the pretreatment may be contained within a distinct domain of a single film, designed to dissolve and release to the mucosa prior to release of the secondary domains with or without penetration enhancer(s) or API(s). The active ingredient may then be delivered from a second treatment, alone or in combination with additional penetration enhancer(s). There may also be a tertiary treatment or domain that delivers additional penetration enhancer(s) and/or at least one API(s) or prodrug(s), either at a different ratio relative to each other or relative to the overall loading of the other treatments. This allows a custom pharmacokinetic profile to be obtained. In this way, the product may have single or multiple domains, with penetration enhancer(s) and API(s) that can vary in mucosal application order, composition, concentration, or overall loading that leads to the desired absorption amounts and/or rates that achieve the intended pharmacokinetic profile and/or pharmacodynamic effect.

The film format can be oriented such that no distinct sides, or such that the film has at least one side of a multiple layer film where the edges are co-terminus (having or meeting at a shared border or limit).

The pharmaceutical composition can be a chewable or gelatin or lyophilized or inhalation based dosage form, spray, gum, gel, cream, tablet, capsule, liquid or film.

Microneedles can be used to enhance the delivery of drugs through the oral mucosa, particularly with the claimed compositions. The microneedles create micron sized pores in the oral mucosa which can enhance the delivery of drugs across the mucosa. Solid, hollow or dissolving microneedles can be fabricated out of suitable materials including, but not limited to, metal, polymer, glass and ceramics. The microfabrication process can include photolithography, silicon etching, laser cutting, metal electroplating, metal electro polishing and molding. Microneedles could be solid which is used to pretreat the tissue and are removed before applying the film. The drug loaded polymer film described in this application can be used as the matrix material of the microneedles itself. These films can have microneedles or micro protrusions fabricated on their surface which will dissolve after forming microchannels in the mucosa through which drugs can permeate.

The term "film" can include films and sheets, in any shape, including rectangular, square, or other desired shape. A film can be any desired thickness and size. In preferred embodiments, a film can have a thickness and size such that it can be administered to a user, for example, placed into the oral cavity of the user. A film can have a relatively thin thickness of from about 0.0025mm to about 0.250mm, or a film can have a somewhat thicker thickness of from about 0.250mm to about1.0mm. For some films, the thickness may be even larger, i.e., greater than about 1.0mm or thinner, i.e., less than about 0.0025mm. A film can be a single layer or a film can be multi-layered, including laminated or multiple cast films. A permeation enhancer and pharmaceutically active component can be combined in a single layer, each contained in separate layers, or can each be otherwise contained in discrete regions of the same dosage form. In certain embodiments, the pharmaceutically active component contained in the polymeric matrix can be dispersed in the matrix. In certain embodiments, the permeation enhancer being contained in the polymeric matrix can be dispersed in the matrix.

Oral dissolving films can fall into three main classes: fast dissolving, moderate dissolving and slow dissolving. Oral dissolving films can also include a combination of any of the above categories. Fast dissolving films can dissolve in about 1 second to about 30 seconds in the mouth, including more than 1 second, more than 5 seconds, more than 10 seconds, more than 20 seconds, or less than 30 seconds. Moderate dissolving films can dissolve in about 1 to about 30 minutes in the mouth including more than 1 minute, more than 5 minutes, more than 10 minutes, more than 20 minutes or less than 30 minutes, and slow dissolving films can dissolve in more than 30 minutes in the mouth. As a general trend, fast dissolving films can include (or consist of) low molecular weight hydrophilic polymers (e.g., polymers having a molecular weight between about 1,000 to 9,000 daltons, or polymers having a molecular weight up to 200,000 daltons). In contrast, slow dissolving films generally include high molecular weight polymers (e.g., having a molecular weight in millions). Moderate dissolving films can tend to fall in between the fast and slow dissolving films.

It can be preferable to use films that are moderate dissolving films. Moderate dissolving films can dissolve rather quickly, but also have a good level of mucoadhesion. Moderate dissolving films can also be flexible, quickly wettable, and are typically non-irritating to the user. Such moderate dissolving films can provide a quick enough dissolution rate, most desirably between about 1 minute and about 20 minutes, while providing an acceptable mucoadhesion level such that the film is not easily removable once it is placed in the oral cavity of the user. This can ensure delivery of a pharmaceutically active component to a user.

The pharmaceutically active component is diazepam.

### Polymeric Matrix

The composition includes a polymeric mucoadhesive matrix. Any desired polymeric matrix may be used, provided that it is orally dissolvable or erodible. The dosage should have enough bioadhesion to not be easily removed and it should form a gel like structure when administered. They can be moderate-dissolving in the oral cavity and particularly suitable for delivery of pharmaceutically active components, although both fast release, delayed release, controlled release and sustained release compositions are also among the various embodiments contemplated.

The pharmaceutical composition film can include dendritic polymers which can include highly branched macromolecules with various structural architectures. The dendritic polymers can include dendrimers, dendronised polymers (dendrigrafted polymers), linear dendritic hybrids, multi-arm star polymers, or hyperbranched polymers.

Hyperbranched polymers are highly branched polymers with imperfections in their structure. However, they can be synthesized in a single step reaction which can be an advantage over other dendritic structures and are therefore suitable for bulk volume applications. The properties of these polymers apart from their globular structure are the abundant functional groups, intramolecular cavities, low viscosity and high solubility. Dendritic polymers have been used in several drug delivery applications. See, e.g., Dendrimers as Drug Carriers: Applications in Different Routes of Drug Administration. J Pharm Sci, VOL. 97, 2008, 123-143.

The dendritic polymers can have internal cavities which can encapsulate drugs. The steric hindrance caused by the highly dense polymer chains might prevent the crystallization of the drugs. Thus, branched polymers can provide additional advantages in formulating crystallizable drugs in a polymer matrix.

Examples of suitable dendritic polymers include poly(ether) based dendrons, dendrimers and hyperbranched polymers, poly(ester) based dendrons, dendrimers and hyperbranched polymers, poly(thioether) based dendrons, dendrimers and hyperbranched polymers, poly(amino acid) based dendrons dendrimers and hyperbranched polymers, poly(arylalkylene ether) based dendrons, dendrimers and hyperbranched polymers, poly(alkyleneimine) based dendrons, dendrimers and hyperbranched polymers, poly(amidoamine) based dendrons, dendrimers or hyperbranched polymers.

Other examples of hyperbranched polymers include poly(amines)s, polycarbonates, poly(ether ketone)s, polyurethanes, polycarbosilanes, polysiloxanes, poly(ester amine)s, poly(sulfone amine)s, poly(urea urethane)s and polyether polyols such as polyglycerols.

A film can be produced by a combination of at least one polymer and a solvent, optionally including other components. The solvent may be water, a polar organic solvent including, but not limited to, ethanol, isopropanol, acetone, or any combination thereof. **In** some embodiments, the solvent may be a non-polar organic solvent, such as methylene chloride. The film may be prepared by utilizing a selected casting or deposition method and a controlled drying process. For example, the film may be prepared through a controlled drying processes, which include application of heat and/or radiation energy to the wet film matrix to form a visco-elastic structure, thereby controlling the uniformity of content of the film. The controlled drying processes can include air alone, heat alone or heat and air together contacting the top of the film or bottom of the film or the substrate supporting the cast or deposited or extruded film or contacting more than one surface at the same time or at different times during the drying process. Some of such processes are described in more detail in U.S. Patent No. 8,765,167 and U.S. Patent No. 8,652,378. Alternatively, the films may be extruded as described in U.S. Patent Publication No. 2005/0037055 A1.

A polymer included in the films may be water-soluble, water-swellable, water-insoluble, or a combination of one or more either water-soluble, water-swellable or water-insoluble polymers. The polymer may include cellulose, cellulose derivatives or gums. Specific examples of useful water-soluble polymers include, but are not limited to, polyethylene oxide, pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragancanth gum, guar gum, acacia gum, arabic gum, polyacrylic acid, methylmethacrylate copolymer, carboxyvinyl copolymers, starch, gelatin, and combinations thereof. Specific examples of useful water-insoluble polymers include, but are not limited to, ethyl cellulose, hydroxypropyl ethyl cellulose, cellulose acetate phthalate, hydroxypropyl methyl cellulose phthalate and combinations thereof. For higher dosages, it may be desirable to incorporate a polymer that provides a high level of viscosity as compared to lower dosages.

As used herein the phrase "water-soluble polymer" and variants thereof refer to a polymer that is at least partially soluble in water, and desirably fully or predominantly soluble in water, or absorbs water. Polymers that absorb water are often referred to as being water-swellable polymers. The materials useful with the present invention may be water-soluble or water-swellable at room temperature and other temperatures, such as temperatures exceeding room temperature. Moreover, the materials may be water-soluble or water-swellable at pressures less than atmospheric pressure. In some embodiments, films formed from such water-soluble polymers may be sufficiently water-soluble to be dissolvable upon contact with bodily fluids.

Other polymers useful for incorporation into the films include biodegradable polymers, copolymers, block polymers or combinations thereof. It is understood that the term "biodegradable" is intended to include materials that chemically degrade, as opposed to materials that physically break apart (i.e., bioerodable materials). The polymers incorporated in the films can also include a combination of biodegradable or bioerodable materials. Among the known useful polymers or polymer classes which meet the above criteria are: poly(glycolic acid) (PGA), poly(lactic acid) (PLA), polydioxanes, polyoxalates, poly(alpha-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy)propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of alpha-amino acids, copolymers of alpha-amino acids and caproic acid, copolymers of alpha-benzyl glutamate and polyethylene glycol, copolymers of succinate and poly(glycols), polyphosphazene, polyhydroxy-alkanoates or mixtures thereof. The polymer matrix can include one, two, three, four or more components.

Although a variety of different polymers may be used, the polymers provide mucoadhesive properties to the film, as well as a desired dissolution and/or disintegration rate. In particular, the time period for which it is desired to maintain the film in contact with the mucosal tissue depends on the type of pharmaceutically active component contained in the composition. Some pharmaceutically active components may only require a few minutes for delivery through the mucosal tissue, whereas other pharmaceutically active components may require up to several hours or even longer. Accordingly, in some embodiments, one or more water-soluble polymers, as described above, may be used to form the film. In other embodiments, however, it may be desirable to use combinations of water-soluble polymers and polymers that are water-swellable, water-insoluble and/or biodegradable, as provided above. The inclusion of one or more polymers that are water-swellable, water-insoluble and/or biodegradable may provide films with slower dissolution or disintegration rates than films formed from water-soluble polymers alone. As such, the film may adhere to the mucosal tissue for longer periods of time, such as up to several hours, which may be desirable for delivery of certain pharmaceutically active components.

### Film Properties

Desirably, an individual film dosage of the pharmaceutical film can have a suitable thickness, and small size, which is between about 0.0625-3 inch by about 0.0625-3 inch. The film size can also be greater than 0.0625 inch, greater than 0.5 inch, greater than 1 inch, greater than 2 inches, about 3 inches, and greater than 3 inches, less than 3 inches, less than 2 inches, less than 1 inch, less than 0.5 inch, less than 0.0625 inch in at least one aspect, or greater than 0.0625 inch, greater than 0.5 inch, greater than 1 inch, greater than 2 inches, or greater than 3 inches, about 3 inches, less than 3 inches, less than 2 inches, less than 1 inch, less than 0.5 inch, less than 0.0625 inch in another aspect. The aspect ratio, including thickness, length, and width can be optimized by a person of ordinary skill in the art based on the chemical and physical properties of the polymeric matrix, the active pharmaceutical ingredient, dosage, enhancer, and other additives involved as well as the dimensions of the desired dispensing unit. The film dosage should have good adhesion when placed in the buccal cavity or in the sublingual region of the user. Further, the film dosage should disperse and dissolve at a moderate rate, most desirably dispersing within about 1 minute and dissolving within about 3 minutes. In some embodiments, the film dosage may be capable of dispersing and dissolving at a rate of between about 1 to about 30 minutes, for example, about 1 to about 20 minutes, or more than 1 minute, more than 5 minutes, more than 7 minutes, more than 10 minutes, more than 12 minutes, more than 15 minutes, more than 20 minutes, more than 30 minutes, about 30 minutes, or less than 30 minutes, less than 20 minutes, less than 15 minutes, less than 12 minutes, less than 10 minutes, less than 7 minutes, less than 5 minutes, or less than 1 minute. Sublingual dispersion rates may be shorter than buccal dispersion rates.

For instance, in some embodiments, the films may include polyethylene oxide alone or in combination with a second polymer component. The second polymer may be another water-soluble polymer, a water-swellable polymer, a water-insoluble polymer, a biodegradable polymer or any combination thereof. Suitable water-soluble polymers include, without limitation, any of those provided above. In some embodiments, the water-soluble polymer may include hydrophilic cellulosic polymers, such as hydroxypropyl cellulose and/or hydroxypropylmethyl cellulose. In some embodiments, one or more water-swellable, water-insoluble and/or biodegradable polymers also may be included in the polyethylene oxide-based film. Any of the water-swellable, water-insoluble or biodegradable polymers provided above may be employed. The second polymer component may be employed in amounts of about 0% to about 80% by weight in the polymer component, more specifically about 30% to about 70% by weight, and even more specifically about 40% to about 60% by weight, including greater than 5%, greater than 10%, greater than 15%, greater than 20%, greater than 30%, greater than 40%, greater than 50%, greater than 60%, and greater than 70%, about 70%, less than 70%, less than 60%, less than 50%, less than 40%, less than 30%, less than 20%, less than 10% or less than 5% by weight.

### Additives

Additives may be included in the films. Examples of classes of additives include preservatives, antimicrobials, excipients, lubricants, buffering agents, stabilizers, blowing agents, pigments, coloring agents, fillers, bulking agents, sweetening agents, flavoring agents, fragrances, release modifiers, adjuvants, plasticizers, flow accelerators, mold release agents, polyols, granulating agents, diluents, binders, buffers, absorbents, glidants, adhesives, anti-adherents, acidulants, softeners, resins, demulcents, solvents, surfactants, emulsifiers, elastomers, anti-tacking agents, anti-static agents and mixtures thereof. These additives may be added with the pharmaceutically active component(s). As used herein, the term "stabilizer" means an excipient capable of preventing aggregation or other physical degradation, as well as chemical degradation, of the active pharmaceutical ingredient, another excipient, or the combination thereof.

Stabilizers may also be classified as antioxidants, sequestrants, pH modifiers, emulsifiers and/or surfactants, or UV stabilizers.

Antioxidants (i.e., pharmaceutically compatible compound(s) or composition(s) that decelerates, inhibits, interrupts and/or stops oxidation processes) include, in particular, the following substances: tocopherols and the esters thereof, sesamol of sesame oil, coniferyl benzoate of benzoin resin, nordihydroguaietic resin and nordihydroguaiaretic acid (NDGA), gallates (among others, methyl, ethyl, propyl, amyl, butyl, lauryl gallates), butylated hydroxyanisole (BHA/BHT, also butyl-p-cresol); ascorbic acid and salts and esters thereof (for example, acorbyl palmitate), erythorbinic acid (isoascorbinic acid) and salts and esters thereof, monothioglycerol, sodium formaldehyde sulfoxylate, sodium metabisulfite, sodium bisulfite, sodium sulfite, potassium metabisulfite, butylated hydroxyanisole, butylated hydroxytoluene (BHT), propionic acid. Typical antioxidants are tocopherol such as, for example, α-tocopherol and the esters thereof, butylated hydroxytoluene and butylated hydroxyanisole. The terms "tocopherol" also includes esters of tocopherol. A known tocopherol is α-tocopherol. The term "α-tocopherol" includes esters of α-tocopherol (for example, α-tocopherol acetate).

Sequestrants (i.e., any compounds which can engage in host-guest complex formation with another compound, such as the active ingredient or another excipient; also referred to as a sequestering agent) include calcium chloride, calcium disodium ethylene diamine tetra-acetate, glucono delta-lactone, sodium gluconate, potassium gluconate, sodium tripolyphosphate, sodium hexametaphosphate, and combinations thereof. Sequestrants also include cyclic oligosaccharides, such as cyclodextrins, cyclomannins (5 or more α-D-mannopyranose units linked at the 1,4 positions by α linkages), cyclogalactins (5 or more β-D-galactopyranose units linked at the 1,4 positions by β linkages), cycloaltrins (5 or more α-D-altropyranose units linked at the 1,4 positions by α linkages), and combinations thereof.

pH modifiers or stabilizers include acids (e.g., tartaric acid, citric acid, lactic acid, fumaric acid, phosphoric acid, ascorbic acid, acetic acid, succinic acid, adipic acid and maleic acid), acidic amino acids (e.g., glutamic acid, aspartic acid, etc.), inorganic salts (alkali metal salt, alkaline earth metal salt, ammonium salt, etc.) of such acidic substances, a salt of such acidic substance with an organic base (e.g., basic amino acid such as lysine, arginine and the like, meglumine and the like), and a solvate (e.g., hydrate) thereof. Other examples of pH modifiers include silicified microcrystalline cellulose, magnesium aluminometasilicate, calcium salts of phosphoric acid (e.g., calcium hydrogen phosphate anhydrous or hydrate, calcium, sodium or potassium carbonate or hydrogencarbonate and calcium lactate or mixtures thereof), sodium and/or calcium salts of carboxymethyl cellulose, cross-linked carboxymethylcellulose (e.g., croscarmellose sodium and/or calcium), polacrilin potassium, sodium and or/calcium alginate, docusate sodium, magnesium calcium, aluminium or zinc stearate, magnesium palmitate and magnesium oleate, sodium stearyl fumarate, and combinations thereof.

Examples of emulsifiers and/or surfactants include poloxamers or pluronics, polyethylene glycols, polyethylene glycol monostearate, polysorbates, sodium lauryl sulfate, polyethoxylated and hydrogenated castor oil, alkyl polyoside, a grafted water soluble protein on a hydrophobic backbone, lecithin, glyceryl monostearate, glyceryl monostearate/polyoxyethylene stearate, ketostearyl alcohol/sodium lauryl sulfate, carbomer, phospholipids, (C₁₀-C₂₀)-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfonates and hydroxyalkanesulfonates, olefinsulfonates, acyl esters of isethionates, α-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, and acylglutamates, quaternary ammonium salts (e.g., di-(C₁₀-C₂₄)-alkyl-dimethylammonium chloride or bromide), (C₁₀-C₂₄)-alkyl-dimethylethylammonium chloride or bromide, (C₁₀-C₂₄)-alkyl-trimethylammonium chloride or bromide (e.g., cetyltrimethylammonium chloride or bromide), (C₁₀-C₂₄)-alkyl-dimethylbenzylammonium chloride or bromide (e.g., (C₁₂-C₁₈)-alkyl-dimethylbenzylammonium chloride), N-(C₁₀-C₁₈)-alkyl-pyridinium chloride or bromide (e.g., N-(C₁₂-C₁₆)-alkyl-pyridinium chloride or bromide), N-(C₁₀-C₁₈)-alkyl-isoquinolinium chloride, bromide or monoalkyl sulfate, N-(C₁₂-C₁₈)-alkyl-polyoylaminoformylmethylpyridinium chloride, N-(C₁₂-C₁₈)-alkyl-N-methylmorpholinium chloride, bromide or monoalkyl sulfate, N-(C₁₂-C₁₈)-alkyl-N-ethylmorpholinium chloride, bromide or monoalkyl sulfate, (C₁₆-C₁₈)-alkyl-pentaoxethylammonium chloride, diisobutylphenoxyethoxyethyldimethylbenzylammonium chloride, salts of N,N-di-ethylaminoethylstearylamide and -oleylamide with hydrochloric acid, acetic acid, lactic acid, citric acid, phosphoric acid, N-acylaminoethyl-N,N-diethyl-N-methylammonium chloride, bromide or monoalkyl sulfate, and N-acylaminoethyl-N,N-diethyl-N-benzylammonium chloride, bromide or monoalkyl sulfate (in the foregoing, "acyl" standing for, e.g., stearyl or oleyl), and combinations thereof.

Examples of UV stabilizers include UV absorbers (e.g., benzophenones), UV quenchers (i.e., any compound that dissipates UV energy as heat, rather than allowing the energy to have a degradation effect), scavengers (i.e., any compound that eliminates free radicals resulting from exposure to UV radiation), and combinations thereof.

In other embodiments, stabilizers include ascorbyl palmitate, ascorbic acid, alpha tocopherol, butylated hydroxytoluene, buthylated hydroxyanisole, cysteine HC1, citric acid, ethylenediamine tetra acetic acid (EDTA), methionine, sodium citrate, sodium ascorbate, sodium thiosulfate, sodium metabi sulfite, sodium bisulfite, propyl gallate, glutathione, thioglycerol, singlet oxygen quenchers, hydroxyl radical scavengers, hydroperoxide removing agents, reducing agents, metal chelators, detergents, chaotropes, and combinations thereof. "Singlet oxygen quenchers" include, but are not limited to, alkyl imidazoles (e.g., histidine, L-camosine, histamine, imidazole 4-acetic acid), indoles (e.g., tryptophan and derivatives thereof, such as N-acetyl-5-methoxytryptamine, N-acetylserotonin, 6-methoxy-1,2,3,4-tetrahydro-beta-carboline), sulfur-containing amino acids (e.g., methionine, ethionine, djenkolic acid, lanthionine, N-formyl methionine, felinine, S-allyl cysteine, S-aminoethyl-L-cysteine), phenolic compounds (e.g., tyrosine and derivatives thereof), aromatic acids (e.g., ascorbate, salicylic acid, and derivatives thereof), azide (e.g., sodium azide), tocopherol and related vitamin E derivatives, and carotene and related vitamin A derivatives. "Hydroxyl radical scavengers" include, but are not limited to azide, dimethyl sulfoxide, histidine, mannitol, sucrose, glucose, salicylate, and L-cysteine. "Hydroperoxide removing agents" include, but are not limited to catalase, pyruvate, glutathione, and glutathione peroxidases. "Reducing agents" include, but are not limited to, cysteine and mercaptoethylene. "Metal chelators" include, but are not limited to, EDTA, EGTA, o-phenanthroline, and citrate. "Detergents" include, but are not limited to, SDS and sodium lauroyl sarcosyl. "Chaotropes" include, but are not limited to guandinium hydrochloride, isothiocyanate, urea, and formamide. As discussed herein, stabilizers can be present in 0.0001%-50% by weight, including greater than 0.0001%, greater than 0.001%, greater than 0.01%, greater than 0.1%, greater than 1%, greater than 5%, greater than 10%, greater than 20%, greater than 30%, greater than 40%, greater than 50%, less than 50%, less than 40%, less than 30%, less than 20%, less than 10%, less than 1%, less than 0.1%, less than 0.01%, less than 0.001%, or less than 0.0001% by weight.

Useful additives can include, for example, gelatin, gelatin hydrosylates, recombinant gelatin, vegetable proteins such as sunflower protein, soybean proteins, cotton seed proteins, peanut proteins, grape seed proteins, whey proteins, whey protein isolates, blood proteins, egg proteins, acrylated proteins, water-soluble polysaccharides such as alginates, carrageenans, guar gum, agar-agar, xanthan gum, gellan gum, gum arabic and related gums (gum ghatti, gum karaya, gum tragancanth), pectin, water-soluble derivatives of cellulose: alkylcelluloses hydroxyalkylcelluloses and hydroxyalkylalkylcelluloses, such as methylcellulose, hydroxymethylcellulose, hydroxyethylcellulose, hydroxypropylcellulose, hydroxyethylmethylcellulose, hydroxypropylmethylcellulose, hydroxybutylmethylcellulose, cellulose esters and hydroxyalkylcellulose esters such as cellulose acetate phthalate (CAP), hydroxypropylmethylcellulose (HPMC); carboxyalkylcelluloses, carboxyalkylalkylcelluloses, carboxyalkylcellulose esters such as carboxymethylcellulose and their alkali metal salts; water-soluble synthetic polymers such as polyacrylic acids and polyacrylic acid esters, polymethacrylic acids and polymethacrylic acid esters, polyvinylacetates, polyvinylalcohols, polyvinylacetatephthalates (PVAP), polyvinylpyrrolidone (PVP), PVA/vinyl acetate copolymer, and polycrotonic acids; also suitable are phthalated gelatin, gelatin succinate, crosslinked gelatin, shellac, water-soluble chemical derivatives of starch, cationically modified acrylates and methacrylates possessing, for example, a tertiary or quaternary amino group, such as the diethylaminoethyl group, which may be quaternized if desired; or other similar polymers.

Stabilizers can include nanoparticulate stabilizers, such as a dispersant layer around a nanoparticulate surface. *See, e.g.,* Langmuir 2007, (23)3, 1081-1090, December 20, 2006, https://doi.org/10.1021/1a062042s. Stabilizers can include stabilizer ligands, e.g., monomers bearing functional groups that can get chemisorbed on nanoparticles to form polymerizable monolayers. *See, e.g.,* Jadhav et al https://doi.org/10.1002/ppsc.201400074. Stabilizers can include surface stabilizers. *See, e.g.,* U.S. Pat. No. 6428814 and Japanese Pat. JP 4598399B2. Surface stabilizers can include tyloxapol (U.S. Pat. No. 5,429,824), polyalkylene block copolymers (U.S. Pat. No. 5,565,188), sulfated non-ionic block copolymers (U.S. Pat. No. 5,569,448), high molecular weight, linear, poly(ethylene oxide) polymers (U.S. Pat. No. 5,580,579), butylene oxide-ethylene oxide block copolymers (U.S. Pat. No. 5,587,143), hydroxypropyl cellulose (U.S. Pat. No. 5,591,456), and sugar based surface stabilizers (U.S. Pat. No. 5,622,938). Stabilizers can include peptide stabilizers. See, e.g., WO2006097748A2. Stabilizers can include for example, L-cysteine hydrochloride, glycine hydrochloride, malic acid, sodium metabisulfite, citric acid, tartaric acid, and L-cystine dihydrochloride. See, e.g., U.S. Pat. 6,153,223. Stabilizers can include natural compounds. Stabilizers can include synthetic compounds. Stabilizers can include a blend of one of more compounds or categories of compounds described above. Stabilizers can be function to protect the metabolism of a prodrug until a desired time or until it reaches a specific target, tissue or environment.

The additional components can range up to about 80%, desirably about 0.005% to 50% and more desirably within the range of 1% to 20% based on the weight of all composition components, including greater than 1%, greater than 5%, greater than 10%, greater than 20%, greater than 30%, greater than 40%, greater than 50%, greater than 60%, greater than 70%, about 80%, greater than 80%, less than 80%, less than 70%, less than 60%, less than 50%, less than 40%, less than 30%, less than 20%, less than 10%, less than 5%, about 3%, or less than 1%. Other additives can include anti-tacking, flow agents and opacifiers, such as the oxides of magnesium aluminum, silicon, titanium, etc. desirably in a concentration range of about 0.005% to about 5% by weight and desirably about 0.02% to about 2% based on the weight of all film components, including greater than 0.02%, greater than 0.2%, greater than 0.5%, greater than 1%, greater than 1.5%, greater than 2%, greater than 4%, about 5%, greater than 5%, less than 4%, less than 2%, less than 1%, less than 0.5%, less than 0.2%, or less than 0.02%.

In certain embodiments, the composition can include plasticizers, which can include polyalkylene oxides, such as polyethylene glycols, polypropylene glycols, polyethylene-propylene glycols, organic plasticizers with low molecular weights, such as glycerol, glycerol monoacetate, diacetate or triacetate, triacetin, polysorbate, cetyl alcohol, propylene glycol, sugar alcohols sorbitol, sodium diethylsulfosuccinate, triethyl citrate, tributyl citrate, phytoextracts, fatty acid esters, fatty acids, oils and the like, added in concentrations ranging from about 0.1% to about 40%, and desirably ranging from about 0.5% to about 20% based on the weight of the composition including greater than 0.5%, greater than 1%, greater than 1.5%, greater than 2%, greater than 4%, greater than 5%, greater than 10%, greater than 15%, about 20%, greater than 20%, less than 20%, less than 15%, less than 10%, less than 5%, less than 4%, less than 2%, less than 1%, or less than 0.5%. There may further be added compounds to improve the texture properties of the film material such as animal or vegetable fats, desirably in their hydrogenated form. The composition can also include compounds to improve the textural properties of the product. Other ingredients can include binders which contribute to the ease of formation and general quality of the films. Non-limiting examples of binders include starches, natural gums, pregelatinized starches, gelatin, polyvinylpyrrolidone, methylcellulose, sodium carboxymethylcellulose, ethylcellulose, polyacrylamides, polyvinyloxoazolidone, or polyvinylalcohols.

Further potential additives include solubility enhancing agents, such as substances that form inclusion compounds with active components. Such agents may be useful in improving the properties of very insoluble and/or unstable actives. In general, these substances are doughnut-shaped molecules with hydrophobic internal cavities and hydrophilic exteriors. Insoluble and/or instable pharmaceutically active components may fit within the hydrophobic cavity, thereby producing an inclusion complex, which is soluble in water. Accordingly, the formation of the inclusion complex permits very insoluble and/or unstable pharmaceutically active components to be dissolved in water. A particularly desirable example of such agents are cyclodextrins, which are cyclic carbohydrates derived from starch. Other similar substances, however, are considered well within the scope of the present invention.

Suitable coloring agents include food, drug and cosmetic colors (FD&C), drug and cosmetic colors (D&C), or external drug and cosmetic colors (Ext. D&C). These colors are dyes, their corresponding lakes, and certain natural and derived colorants. Lakes are dyes absorbed on aluminum hydroxide. Other examples of coloring agents include known azo dyes, organic or inorganic pigments, or coloring agents of natural origin. Inorganic pigments are preferred, such as the oxides or iron or titanium, these oxides, being added in concentrations ranging from about 0.001 to about 10%, and preferably about 0.5 to about 3%, including greater than 0.001%, greater than 0.01%, greater than 0.1%, greater than 0.5%, greater than 1%, greater than 2%, greater than 5%, about 10%, greater than 10%, less than 10%, less than 5%, less than 2%, less than 1%, less than 0.5%, less than 0.1%, less than 0.01%, or less than 0.001%, based on the weight of all the components.

Flavors may be chosen from natural and synthetic flavoring liquids. An illustrative list of such agents includes volatile oils, synthetic flavor oils, flavoring aromatics, oils, liquids, oleoresins or extracts derived from plants, leaves, flowers, fruits, stems and combinations thereof. A non-limiting representative list of examples includes mint oils, cocoa, and citrus oils such as lemon, orange, lime and grapefruit and fruit essences including apple, pear, peach, grape, strawberry, raspberry, cherry, plum, pineapple, apricot or other fruit flavors. Other useful flavorings include aldehydes and esters such as benzaldehyde (cherry, almond), citral i.e., alphacitral (lemon, lime), neral, i.e., beta-citral (lemon, lime), decanal (orange, lemon), aldehyde C-8 (citrus fruits), aldehyde C-9 (citrus fruits), aldehyde C-12 (citrus fruits), tolyl aldehyde (cherry, almond), 2,6-dimethyloctanol (green fruit), or 2-dodecenal (citrus, mandarin), combinations thereof and the like.

The sweeteners may be chosen from the following non-limiting list: saccharides, glucose (corn syrup), dextrose, invert sugar, fructose, and combinations thereof, saccharin and its various salts such as the sodium salt; dipeptide based sweeteners such as aspartame, neotame, advantame; dihydrochalcone compounds, glycyrrhizin; Stevia Rebaudiana (Stevioside); chloro derivatives of sucrose such as sucralose; sugar alcohols such as sorbitol, mannitol, xylitol, and the like. Also contemplated are hydrogenated starch hydrolysates and the synthetic sweetener 3,6-dihydro-6-methyl-1-1-1,2,3-oxathiazin-4-one-2,2-dioxide, particularly the potassium salt (acesulfame-K), and sodium and calcium salts thereof, and natural intensive sweeteners, such as Lo Han Kuo. Other sweeteners may also be used.

Anti-foaming and/or de-foaming components may also be used with the films. These components aid in the removal of air, such as entrapped air, from the film-forming compositions. Such entrapped air may lead to non-uniform films. Simethicone is one particularly useful anti-foaming and/or de-foaming agent. The present invention, however, is not so limited and other suitable anti-foam and/or de-foaming agents may be used. Simethicone and related agents may be employed for densification purposes. More specifically, such agents may facilitate the removal of voids, air, moisture, and similar undesired components, thereby providing denser and thus more uniform films. Agents or components which perform this function can be referred to as densification or densifying agents. As described above, entrapped air or undesired components may lead to non-uniform films.

Any other optional components described in commonly assigned U.S. Patent No. 7,425,292 and U.S. Patent No. 8,765,167, referred to above, also may be included in the films described herein.

The film compositions further desirably contain a buffer so as to control the pH of the film composition. Any desired level of buffer may be incorporated into the film composition so as to provide the desired pH level encountered as the pharmaceutically active component is released from the composition. The buffer is preferably provided in an amount sufficient to control the release from the film and/or the absorption into the body of the pharmaceutically active component. In some embodiments, the buffer may include sodium citrate, citric acid, bitartrate salt and combinations thereof.

The pharmaceutical films described herein may be formed via any desired process. Suitable processes are set forth in U.S. Patent Nos. 8,652,378, 7,425,292 and 7,357,891. In one embodiment, the film dosage composition is formed by first preparing a wet composition, the wet composition including a polymeric carrier matrix and a therapeutically effective amount of a pharmaceutically active component. The wet composition is cast into a film and then sufficiently dried to form a self-supporting film composition. The wet composition may be cast into individual dosages, or it may be cast into a sheet, where the sheet is then cut into individual dosages.

The pharmaceutical composition can adhere to a mucosal surface. The composition carries a pharmaceutical (diazepam), and upon application and adherence to the mucosal surface, offers a layer of protection and delivers the pharmaceutical to the treatment site, the surrounding tissues, and other bodily fluids. The composition provides an appropriate residence time for effective drug delivery at the treatment site, given the control of erosion in aqueous solution or bodily fluids such as saliva, and the slow, natural erosion of the film concomitant or subsequent to the delivery.

The residence time of the composition depends on the erosion rate of the water erodible polymers used in the formulation and their respective concentrations. The erosion rate may be adjusted, for example, by mixing together components with different solubility characteristics or chemically different polymers, such as hydroxyethyl cellulose and hydroxypropyl cellulose; by using different molecular weight grades of the same polymer, such as mixing low and medium molecular weight hydroxyethyl cellulose; by using excipients or plasticizers of various lipophilic values or water solubility characteristics (including essentially insoluble components); by using water soluble organic and inorganic salts; by using crosslinking agents such as glyoxal with polymers such as hydroxyethyl cellulose for partial crosslinking; or by post-treatment irradiation or curing, which may alter the physical state of the film, including its crystallinity or phase transition, once obtained. These strategies might be employed alone or in combination in order to modify the erosion kinetics of the film. Upon application, the pharmaceutical composition film adheres to the mucosal surface and is held in place. Water absorption softens the composition, thereby diminishing the foreign body sensation. As the composition rests on the mucosal surface, delivery of the drug occurs. Residence times may be adjusted over a wide range depending upon the desired timing of the delivery of the chosen pharmaceutical and the desired lifespan of the carrier. Generally, however, the residence time is modulated between about a few seconds to about a few days. Preferably, the residence time for most pharmaceuticals is adjusted from about 5 seconds to about 24 hours. More preferably, the residence time is adjusted from about 5 seconds to about 30 minutes. In addition to providing drug delivery, once the composition adheres to the mucosal surface, it also provides protection to the treatment site, acting as an erodible bandage. Lipophilic agents can be designed to slow down erodibility to decrease disintegration and dissolution.

It is also possible to adjust the kinetics of erodability of the composition by adding excipients which are sensitive to enzymes such as amylase, very soluble in water such as water soluble organic and inorganic salts. Suitable excipients may include the sodium and potassium salts of chloride, carbonate, bicarbonate, citrate, trifluoroacetate, benzoate, phosphate, fluoride, sulfate, or tartrate. The amount added can vary depending upon how much the erosion kinetics is to be altered as well as the amount and nature of the other components in the composition.

Emulsifiers typically used in the water-based emulsions described above are, preferably, either obtained in situ if selected from the linoleic, palmitic, myristoleic, lauric, stearic, cetoleic or oleic acids and sodium or potassium hydroxide, or selected from the laurate, palmitate, stearate, or oleate esters of sorbitol and sorbitol anhydrides, polyoxyethylene derivatives including monooleate, monostearate, monopalmitate, monolaurate, fatty alcohols, alkyl phenols, allyl ethers, alkyl aryl ethers, sorbitan monostearate, sorbitan monooleate and/or sorbitan monopalmitate.

The amount of pharmaceutically active component (diazepam) to be used depends on the desired treatment strength and the composition of the layers, although preferably, the pharmaceutical component comprises from about 0.001% to about 99%, more preferably from about 0.003 to about 75%, and most preferably from about 0.005% to about 50% by weight of the composition, including, more than 0.005%, more than 0.05%, more than 0.5%, more than 1%, more than 5%, more than 10%, more than 15%, more than 20%, more than 30%, about 50%, more than 50%, less than 50%, less than 30%, less than 20%, less than 15%, less than 10%, less than 5%, less than 1%, less than 0.5%, less than 0.05%, or less than 0.005%. The amounts of other components may vary depending on the drug or other components but typically these components comprise no more than 50%, preferably no more than 30%, and most preferably no more than 15% by total weight of the composition.

The thickness of the film may vary, depending on the thickness of each of the layers and the number of layers. As stated above, both the thickness and amount of layers may be adjusted in order to vary the erosion kinetics. Preferably, if the composition has only two layers, the thickness ranges from 0.005 mm to 2 mm, preferably from 0.01 to 1 mm, and more preferably from 0.1 to 0.5 mm, including greater than 0.1 mm, greater than 0.2 mm, about 0.5 mm, greater than 0.5 mm, less than 0.5 mm, less than 0.2 mm, or less than 0.1 mm. The thickness of each layer may vary from 10 to 90% of the overall thickness of the layered composition, and preferably varies from 30 to 60%, including greater than 10%, greater than 20%, greater than 30%, greater than 40%, greater than 50%, greater than 70%, greater than 90%, about 90%, less than 90%, less than 70%, less than 50%, less than 40%, less than 30%, less than 20%, or less than 10%. Thus, the preferred thickness of each layer may vary from 0.01 mm to 0.9 mm, or from 0.03 to 0.5 mm.

### EXAMPLES

The proposed dosing regimen for diazepam buccal film (DBF) was designed to provide diazepam exposure in patients equivalent to the exposure achieved when the reference drug, Diastat Rectal Gel (DRG), which is administered according to its approved product label (Table 1 below). The Diastat label provides a weight-adjusted dosing regimen that categorizes patients first according to three age groups: 2-5 years, 6-11 years, and 12+ years. The recommended dose on mg/kg basis is approximately 0.5 mg/kg, 0.3 mg/kg, and 0.2 mg/kg for the three age groups, respectively. Within each age group, the recommended dose for an individual patient is determined based on 7 weight categories.

**Table 1 Dosing Regimen for Diastat Rectal Gel (DRG) According to Label**

| **2** - **5 Years** | | **6 - 11 years** | | **12+ Years** | |
|---|---|---|---|---|---|
| 0.5 mg/kg | | 0.3 mg/kg | | 0.2 mg/kg | |
| Weight (kg) | Dose (mg) | Weight (kg) | Dose (mg) | Weight (kg) | Dose (mg) |
| 6 to 10 | 5 | 10 to 16 | 5 | 14 to 25 | 5 |
| 11 to 15 | 7.5 | 17 to 25 | 7.5 | 26 to 37 | 7.5 |
| 16 to 20 | 10 | 26 to 33 | 10 | 38 to 350 | 10 |
| 21 to 25 | 12.5 | 34 to 41 | 12.5 | 51 to 62 | 12.5 |
| 26 to 30 | 15 | 42 to 50 | 15 | 63 to 75 | 15 |
| 31 to 35 | 17.5 | 51 to 58 | 17.5 | 76 to 87 | 17.5 |
| 36 to 44 | 20 | 59 to 74 | 20 | 88 to 111 | 20 |

Invention of an appropriate dosing regimen for DBF was equivalent to creating a mapping such that an appropriate DBF dose in mg could be specified for any patient based on age group and weight category in the Diastat label. The appropriate dose of DBF would be that dose expected to provide equivalent exposure to diazepam as the exposure provided by labeled dose of Diastat. The initial emphasis of this effort was to create this mapping for adult age group (patients age 12 and older). This effort was complicated by two observations that differentiated DBF from DRG: (1) The pharmacokinetics of DBF were linear. For DBF, both Cmax and AUC were proportional to the dose, whereas for DRG the Cmax was less than proportional to the dose. (2) DBF exhibited a food effect. Cmax for DBF was reduced on average by approximately 33% following a moderate fat meal, and on average approximately 45% following a high-fat meal with no effect on AUC. It was assumed that DRG, because of its rectal administration, was not subject to a significant food effect. The approved label for DRG (Diastat rectal gel) does not report a food effect study and no information pertinent to any effect of food is provided in the Diastat label.

### Example 1 - Two Pilot Studies in Healthy volunteers

As shown in **Figure 1A****,** to span the dosage range indicated in Table 1, two formulations were initially developed. The lower dose formulation utilized dose proportionality via size to produce the 5mg, 7.5mg and 10mg strengths. The higher dose formulation also used dose proportionality via size to produce the 12.5mg, 15mg, 17.5mg, and 20mg strengths. The high and low dose formulations were tested in two pilot clinical crossover studies against diastat rectal gel at at doses of 5mg and 20mg.

The results showed excellent agreement for the PK parameters Cmax and AUC between the 5mg DBF and the 5mg rectal gel. The data also supported dose proportionality for DRG over the dose range 5 to 20 mg), but suggested that DRG was not dose-proportional. For DRG, the increase in the Cmax between the 5 mg and the 20 mg dose was less than dose-proportional. Results for these pilot studies are summarized in Table 2.

**Table 2**

| Reported Mean PK Parameters | | | | |
|---|---|---|---|---|
| | 5mg buccal | 20mg buccal | 5mg rectal | 20mg rectal |
| Tmax (h) | 0.6937 | 1.2083 | 0.3497 | 1.0059 |
| half life (h) | 84.6575 | 81.9649 | 71.4004 | 81.0029 |
| Auct (ng.h/mL) | 3409.4558 | 16104.518 | 3475.1045 | 15358.5481 |
| auc inf | 4415.2048 | 18338.9617 | 4160.7298 | 17713.9903 |
| Cmax (ng/mL) | 178.0456 | 673.5343 | 166.352 | 424.3443 |

| Comparison: Ratio of the Parameters as indicated | | | | |
|---|---|---|---|---|
| | 20 vs 5 buccal | 20 vs 5 rectal | 5b vs 5r | 20b vs 20r |
| Tmax | 1.742 | 2.876 | 1.984 | 1.201 |
| half life | 0.968 | 1.134 | 1.186 | 1.012 |
| Auct | 4.723 | 4.420 | 0.981 | 1.049 |
| auc inf | 4.154 | 4.257 | 1.061 | 1.035 |
| Cmax | 3.783 | 2.551 | 1.070 | 1.587 |

Based on the results of these pilot studies (the observation that mean Cmax following 20 mg DBF was approximately 58.7% higher than Cmax following 20 mg DRG) the formulation development plan was revised as shown in Table 3 below. The available data suggested that a DBF dose of approximately 12.5 mg would produce approximately equal to the Cmax from DRG dose of 20 mg (the highest marked dose). Therefore, the high dose formulation not necessary. In this revised formulation development plan, all dosages are produced from a single formulation (formerly the low dose formulation).

**Table 3 Revised Formulation Development Plan**

| **Description of DBSF Doses** | | |
|---|---|---|
| **DBSF Strength (mg)** | **Film Dimension (mm)** | **Nominal Film Weight (mg)** |
| 5 | 12.8 × 22.0 | 45.0 |
| 6 | 15.4 × 22.0 | 54.0 |
| 7 | 17.9 × 22.0 | 62.9 |
| 8 | 20.5 × 22.0 | 71.9 |
| 9 | 23.0 × 22.0 | 80.9 |
| 10 | 25.6 × 22.0 | 90.0 |
| 12.5 | 32 × 22.0 | 112.5 |

The dose proportionality of DBF was formally investigated in a crossover study in healthy volunteers at doses of 5mg, 10mg and 15mg. A 15mg dose was included as the top dose in the proportionality study to establish linearity and provide flexibility as a potential titration dose. This study demonstrated dose-proportionality for both Cmax and AUC as shown in Figure 1A and Figure 1 B.

In view of these results, the next study was a crossover to provide a direct comparison of the pharmacokinetics of DBF and DRG. This four-treatment, four-period crossover compared one DBF dose (15mg) against three doses (5mg, 12.5mg, and 20mg) of the rectal gel. Because DBF had been shown to be dose-proportional, one dose level for DBF was sufficient. The purpose of this pivotal comparison was to investgate the relationship between DBF and DRG exposures (both Cmax and AUC) over the DRG dosing range (5 - 20 mg). The study design also allowed for formal investigation of dose-proportionality for DRG .

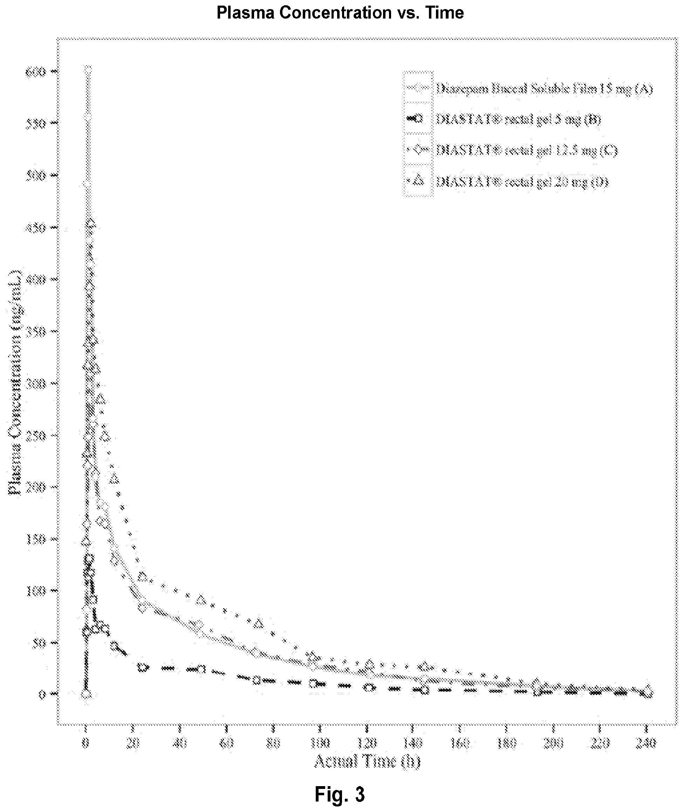

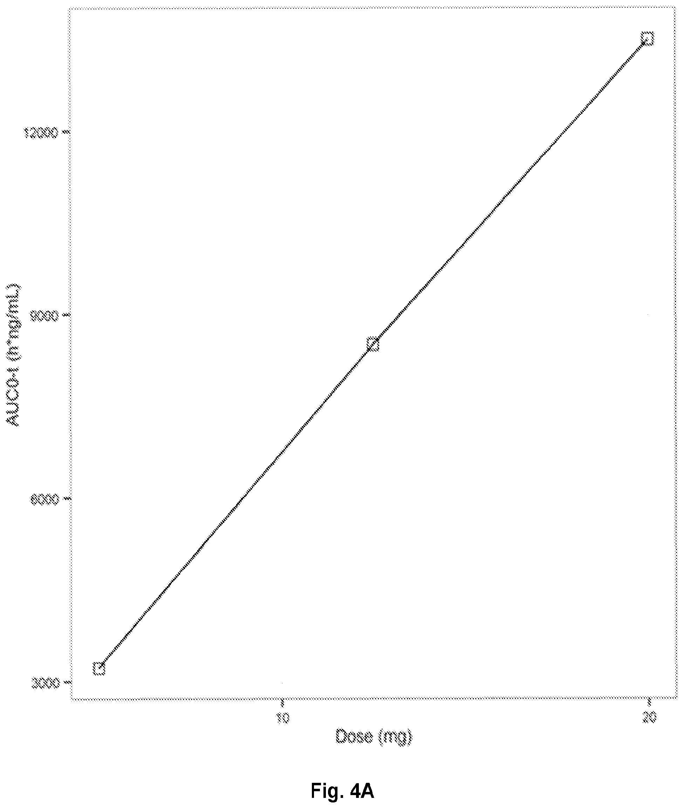

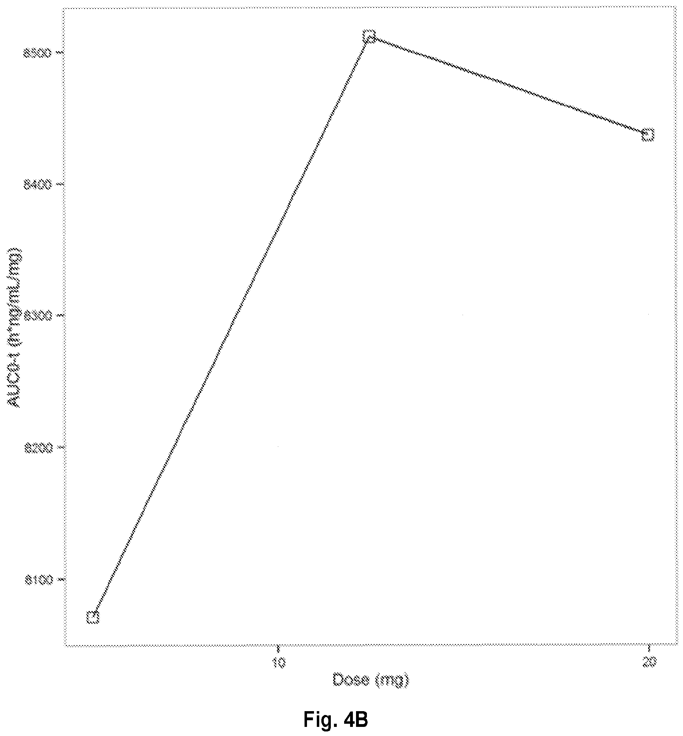

Results of this study for Cmax are shown in Figure 1C. Plasma concentration-time curves for all treatments for a typical subject subject are shown in Figure 3. This study demsonstrated formally that Cmax following DRG was less than dose-proportional (Figure 4B), demonstrated the AUC following DRG was approximately dose-proportional (Figure 4A), and demonstrated that the relative bioavailability of DBF was approximately 118% compared to DRG. The study also permitted estimation of the ratios of PK parameters Cmax and AUC between the formulations comparing these parameters for the studied doses of DRG (5mg, 12.5, 20 mg) with any dose of DBF. These comparisons for the studied doses of DRG were immmediately feasible because DBF was demonstrated to be dose-proportional. Comparisons across all possible doses of DRG with all possible doses of DBF were subsequently facilitated by population PK methods as described in other sections of this document.

### Food Effect Studies

The applicants conducted two food effect studies with DBF: a two arm crossover to investigate the effect of a standard high fat meal, and a four-arm crossover to investigate the effect of position (upright or reclining) under fasting conditions and the effect of a standard moderate fat meal and a standard high fat meal under reclining conditions. The two-arm food effect study showed that a high fat meal taken within 30 minutes of administration reduced Cmax on average by approximately 45% with no effect on AUC. The four-arm study showed that position (whether upright or reclining) had no effect on diazepam PK. The effect of a high fat meal (reclining condition) in the four-arm study was in close agreement with the effect observed in the two-arm study. The moderate fat meal taken within 30 minutes of administration reduced Cmax on average by approximately 33% with no effect on AUC. Food was also associated with a delay in Tmax. Median Tmax fasted was approximately 1 hour whereas median Tmax under fed conditions was 2-3 h. (By comparison Tmax following administration of Diastat as reported in the Diastat label is 1.5 h.)

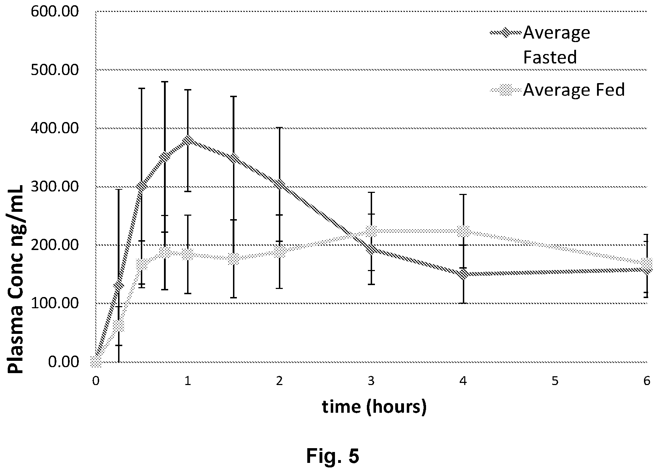

**Figure 5** displays mean plasma concentration-time curves from the two-arm crossover (N=18). Per the Valium label, Diazepam, taken orally has a reduction in exposure following a moderate fat meal. A 20% reduction in Cmax, and a 27% reduction in AUC was reported along with an up to 2.5 hr shift in Tmax. Accordingly, when conducting the food effect study for DBF, it was anticipated that the portion of the drug swallowed, would then be subject to a food effect as well. However, surprisingly, the study showed that the unique attributes of the formulation resulted in a different food effect when compared to oral Valium. Following the administration of a high fat meal, DBF demonstrated a food effect that had a 47% reduction in Cmax, but no reduction in AUC. This was clearly unexpected and significantly greater than what was reported in the literature for Cmax associated with oral Valium, while differing from what was reported in the literature for AUC for oral Valium.

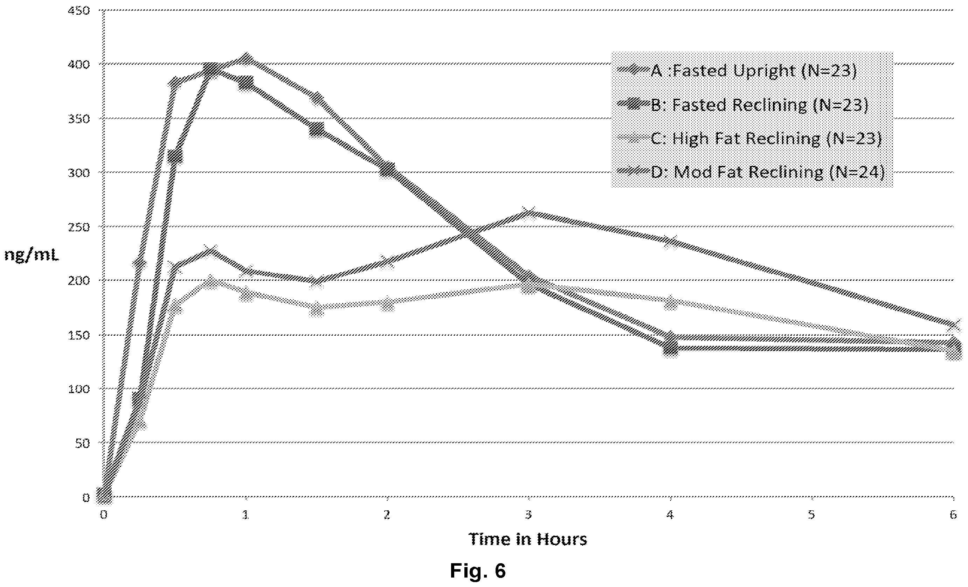

**Figure 6** displays mean plasma concentration-time curves from the four-arm crossover (N=24). This study sought to determine the effect of moderate fat meal on DBF absorption. Another objective of this study was to determine if the administration procedure could be altered to drive more transmucosal absorption. An increase in the early (transmucosal) portion of the profile might then be sufficient to reduce the Tmax. To achieve this a second fasting arm was added.

In prior studies with DBF, DBF was administered with the subjects sitting upright with the film applied to the buccal mucosa for a period of 5 min. At this time, they swallowed any remaining drug product. To drive further absorption, DBF was administered with the subject reclining on his or her side with the film placed the film on the lower buccal mucosa (such that all saliva pooled to the sight of administration). With subjects in this reclining position, DBF was administered in crossover fashion under conditions of fasting, a moderate fat meal, and high fat meal. The time that subjects were requested to swallow was increased from 5 min to 15 min to increase residence time. (A fourth treatment - administration fasting in an upright position was added as a control.) Residence time is routinely reported as one of three key drivers for transmucosal absorption (the others being surface area and permeation kinetics). As shown in Figure 6, position (upright vs. reclining) had no impact under the fasting condition. The effect of the high fat meal in this study was nearly identical to the effect of a high fat meal observed in the two-arm study where DBF was administered so subjects in an upright position. Thus, the changes in the mode of administration aimed at increasing residence time had no apparent effect in either the fasting or the fed condition. The moderate fat meal served only to increase the height of the second portion of the bimodal profile compared with the high fat meal. This provided further evidence that a separation in time between the transbuccal absorption and absorption from the GI tract after food provided an explanation for the later Tmax observed under fed conditions.. It should be noted that following the moderate fat meal, the Cmax was reduced by ~33%, significantly less than the ~45% reduction observed following a high fat meal.

### Population Pharmacokinetic Modeling

Pharmacokinetic (PK) studies in healthy volunteers demonstrated that DBF was not bioequivalent to DRG. DBF differed from DRG in the following respects (1) DBF exhibited higher bioavailability than DRG; (2) The PK behavior of DBF was linear. Specifically, for DBF both Cmax and AUC increased in proportion to the dose. In contrast, the PK behavior of DRG was not linear. Specifically, for DRG, Cmax increased with dose to a degree that was less than dose-proportional, whereas AUC increased in proportion to the dose. (3) DBF exhibited a food effect (~45% reduction on average in Cmax after a high fat meal and ~33% reduction on average after a moderate fat meal with no change in AUC). In contrast, it is assumed that DRG, because of its rectal route of administration, is not affected by food.

Accordingly, Aquestive used population PK modeling to select a dosing regimen to compensate for the differences in PK between DBF and DRG (Table 4). In brief, the recommended DBF dose corresponding to each adult weight class as defined in the Diastat rectal gel label was selected (1) to provide a dose sufficiently high to ensure that the predicted median of the resulting diazepam Cmax following a moderate fat meal was similar to the median Cmax following the labeled dose of Diastat rectal gel, and (2) to provide a dose for which the predicted median of the resulting diazepam Cmax under fasting conditions would not exceed the median Cmax values observed and demonstrated as safe in Phase 1 healthy volunteer studies with DBF. Simulations based on population PK modeling demonstrated that under conditions of a moderate fat meal, the proposed DBF dosing regimen produced for each weight class a Cmax similar to the Cmax expected following the labeled dose of Diastat rectal gel.

**Table 4 DBF Dosing Algorithm**

| **Weight (Kg)** | **DRG (mg)*** | **DBF Weight - Adjusted (mg)**** |
|---|---|---|
| 14 to 25 | 5 | 5 |
| 26 to 37 | 7.5 | 7.5 |
| 38 to 50 | 10 | 10* |
| 51 to 62 | 12.5 | 12.5* |
| 63 to 75 | 15 | 15* |
| 76 to 87 | 17.5 | 15* |
| 88 to 111 | 20 | 17.5* |

| | | |
|---|---|---|
| *The performance of this dosing regimen was evaluated in a clinical study (DBF Crossover with Diastat (DRG) in Patients with Epilepsy) reported below. | | |

### EMU Study

A study was conducted in patients with epilepsy to determine whether the pharmacokinetics of diazepam administered as DBF were changed when DBF was given to patients in the interical state (not having seizures) vs. the ictal/periictal state (during or within 5 minutes of cessation of a seizure). This study was conducted with a fixed dose of DBF (12.5 mg to all patients) independent of weight. This study did not use the weight-adjusted dose regimen shown in Table 5.

Applicants measured pharmacokinetic parameters from a single-dose, crossover study in which plasma samples for determination of diazepam concentrations. The following Cmax, AUC and Tmax values were obtained following administration of 12.5 mg DBF in adults with epilepsy.

**Table 5**

| **Parameter** | **Interictal (A)** | **Periictal (B)** | **Ratio B/A (%)** | **90% CI (%)** |
|---|---|---|---|---|
| Cₘₐₓ (ng/mL) | 190.3 | 180.0 | 95.5 | 73.3-121.9 |
| AUC₀₋₄ₕ (h•ng/mL) | 483.8 | 433.3 | 89.6 | 69.2-115.9 |
| Tₘₐₓ (h) | 0.77 | 0.53 | | |

As shown from the above data, pharmacokinetic parameters were derived from a single-dose, crossover study in which plasma samples for determination of diazepam concentrations were drawn at various times up to 4 h after administration of 12.5 mg DBF either when no seizure activity had been observed in the preceding 3 h (interictal) or within 5 min of a seizure (periictal). The study subjects were 35 adult men and women ages 17-65 with poorly controlled tonic-clonic seizures or focal seizures with impaired awareness. Patients were excluded from analysis if both treatments were not completed (4 subjects), critical time points were missing (6 subjects), pre-dose diazepam concentrations were >5% of the subsequent Cₘₐₓ (2 subjects), or DBF was administered in a manner contrary to instructions (5 subjects). Cₘₐₓ and AUC₀₋₄ₕ values are geometric means; Tₘₐₓ values are median values. 90% geometric confidence interval (CI) values were determined using ln-transformed data. Difference in Tₘₐₓ is not significant, p=0.5708 (Wilcoxon signed-rank test). Values shown represent data from 18 evaluable subjects. AUC₀₋₄ₕ, area under the plasma concentration-time curve from 0 to 4 h after dosing; Cₘₐₓ, maximum plasma drug concentration; Tₘₐₓ, time to reach maximum plasma concentration. From Rogawski et al. See, e.g., Rogawski MA, Gong H, Liow K, Aboumatar S, Klein P, Gelfand MA, Jung C, Wargacki S, Mehta R, Heller AH. Pharmacokinetics of diazepam buccal soluble film in adult patients with epilepsy: comparison of bioavailability with periictal and interictal administration, Abstract 2.453, American Epilepsy Society Annual Meeting, www.aesnet.org, 2018.

### Usability Study

A usability evaluation was conducted within the EMU study. The following outcome was measured.

**Table 6**

| **Outcome** | **Interictal (A)** | **Periictal (B)** |
|---|---|---|
| Successful placement of film | 33 (100%) | 33 (100%) |
| Required more than 1 film placement attempt | 0 (0%) | 2 (6.1%) |
| Spit or blew out film | 0 (0%) | 3 (9.1%) |
| Swallowed film | 2 (6.1%) | 1 (3.0%) |

Results of usability evaluation from interictal-perictal crossover study described above. Values indicate number of subjects out of 33; percentages are given in parentheses. From Jung et al. Jung C, Dubow J, Gong H, Liow K, Klein P, Gelfand MA, Wargacki S, Mehta R, Rogawski MA, Heller AH. The usability of diazepam buccal soluble film as an oral treatment in adult patients with epilepsy, Abstract 3.468, American Epilepsy Society Annual Meeting, www.aesnet.org, 2018.

This study showed that exposure to diazepam after DBF in patients was consistent independent of whether patients were dosed at the time of a seizure. Subjects in this study (patients with epilepsy) exhibited lower plasma concentrations than healthy volunteers after adjustment for dose received. Lower plasma concentrations in patients with epilepsy associated with higher clearance of diazepam is well known from the literature, fully expected, and attributed to the effect of heaptic enzyme induction from concomitant antiseizure drugs taken by the patients (reference Dhillon and Richens, 1981). A subsequent study in patients (described below) confirmed that same effect of the same magnitude was observed in patients given DRG.

### DBF Crossover with Diastat (DRG) in Patients with Epilepsy

Applicants tested the performance of the proposed DBF dosing regimen (Table 7-8) in a head to head two-period crossover comparison with Diastat in patients. The primary objective was to compare the PK performance of DBF administered after a moderate fat meal with Diastat (DRG) administered after a moderate fat meal. DBF was administered according to the proposed weight-adjusted dosing regimen in Table 4 and Diastat (DRG) was administered according to the dosing regimen for Diastat in the FDA-approved label. In addition, patients could enroll in an optional third period to receive DBF after a high-fat meal. A secondary objective of the study was to compare the PK performance of DBF administered after a high fat meal with Diastat (DRG) administered after a moderate fat meal. Results of the primary comparison are shown in the Table 7, below.

The ratio [DBF/DRG] of the Cmax values in this head-to-head study (geometric means) was 96.70% with 90% CI 70.53-132.58 % (Table 7 demonstrating successfully that the diazepam Cmax following a moderate fat meal was similar to the Cmax following the labeled dose of DRG (an outcome consistent with predictions from population PK modeling). This result serves to validate the proposed DBF dosing algorithm. Note also that the AUC values (ratio [DBF/DRG] of geometric means for AUC(0-inf) was greater than 100%, despite equal or lower mg doses for DBF, are also consistent with the results of population PK modelling indicating that DBF exhibits higher bioavailability than DRG.

**Table 7: Pharmacokinetic Parameters Following DBF and DRG Administered to Adults with Epilepsy According to Body Weight Following a Moderate-Fat Meal**

| | **DBF (moderate fat)** | **DRG (moderate fat)** | **Ratio of Geometric Means DBF/DRG (%)¹** | **90% CI (%)²** |
|---|---|---|---|---|
| | **Geometric Mean** | **Geometric Mean** | | |
| **Overall (N=28)** | | | | |
| Cₘₐₓ (ng/mL) | 204.26 | 211.22 | 96.70 | 70.53-132.58 |
| AUC_{(0-T)} (ng•h/mL)* | 7290.40 | 5682.09 | 128.31 | 95.93-171.61 |
| AUC_{(0-INF)} (ng•h/mL)* | 8672.09 | 6880.96 | 126.03 | 103.67-153.21 |
| | Median | Median | | |
| Tₘₐₓ (h) | 1.0 | 0.517 | * | |
| Range | 0.483-4.00 | 0.483-2.983 | | |
| *N=27 | | | | |

| **Cₘₐₓ By Weight Group** | | | | |
|---|---|---|---|---|
| **Wt 51-62 kg (n=6)** | | | | |
| Cₘₐₓ (ng/mL) | 258.38 | 358.06 | 72.16 | 51.17-101.76 |
| Dose (mg) | 12.5 | 12.5 | | |
| | | | | |

| **Wt 63-75 kg (n=4)** | | | | |
|---|---|---|---|---|
| Cₘₐₓ (ng/mL) | 234.45 | 258.88 | 90.56 | 27.89-295.09 |
| Dose (mg) | 15.0 | 15.0 | | |
| | | | | |

| **Wt 76-87 kg (n=7)** | | | | |
|---|---|---|---|---|
| Cₘₐₓ (ng/mL) | 201.39 | 293.00 | 68.74 | 46.77-101.01 |
| Dose (mg) | 15.0 | 17.5 | | |
| | | | | |

| **Wt 88-111 kg (n=11)**** | | | | |
|---|---|---|---|---|
| Cₘₐₓ (ng/mL) | 175.56 | 115.82 | 151.58 | 71.59-320.94 |
| Dose (mg) | 17.5 | 20 | | |
| ¹Calculated using least-square means according to the formula e^{(Difference)} × 100. | | | | |
| ² 90% geometric confidence interval using In-transformed data. | | | | |
| *Statistically significant, *P*<0.05. | | | | |
| **The highest weight category included 4 individuals with body weight 112-124.5 kg. | | | | |

The proposed DBF dose regimen also performed well within each weight category and yielded Cmax values more consistent across the weight categories than those following administration of Diastat.

Results of the secondary comparison (the comparison of DBF following a high fat meal with DRG) are shown in Table 8. The ratio [DBF (high-fat)/DRG] of the Cmax values (geometric means) was 82.67% with 90% CI 55.61-122.91 %. This ratio (~ 82.5%) was consistent with the value predicted by population PK modelling and also serves to validate the proposed DBF dosing algorithm.

**Table 8: Pharmacokinetic Parameters Following DBF and DRG Administered to Adults with Epilepsy According to Body Weight Following a High-Fat Meal (DBF) and a Moderate Fat Meal (DRG)**

| | **DBF (high fat)** | **DRG (moderate fat)** | **Ratio of Geometric Means DBF/DRG (%)¹** | **90% CI (%)²** |
|---|---|---|---|---|
| | **Geometric Mean** | **Geometric Mean** | | |
| **Overall (N=9)** | | | | |
| Cₘₐₓ (ng/mL) | 181.61 | 219.67 | 82.67 | 55.61-122.91 |
| AUC_{(0-T)} (ng•h/mL) | 8288.14 | 7663.19 | 108.16 | 81.15-142.92 |
| AUC_{(0-INF)} (ng•h/mL) | 9497.63 | 8430.60 | 112.66 | 86.19-147.24 |
| | Median | Median | | |
| Tₘₐₓ (h) | 1.517 | 0.50 | * | |
| Range | 0.5 - 4.0 | 0.5 - 1.0 | | |
| ¹Calculated using least-square means according to the formula e^{(Difference)} × 100. | | | | |
| ² 90% geometric confidence interval using In-transformed data. | | | | |
| *Statistically significant, *P*<0.05. | | | | |
| **The highest weight category included 4 individuals with body weight 112-124.5 kg. | | | | |

The observed concentration profiles from studies with DBF show that the onset of the absorption profile does not significantly change with food but a clear bimodal absorption profile is seen. As the larger of the two absorption profiles appears to come from the oral portion of the profile, T max is also shifted out more significantly than would be predicted if the entire dose were oral and a higher concentration was present to drive absorption. To verify this hypothesis and to attempt to quantify the amount of transmucosal delivery achieved during the administration of DBF, mathematical peak deconvolution was utilized to describe each of the modes of absorption in the observed profiles. The profiles can then be analyzed individually and because of the high bioavailability resulting from either route of absorption, an estimate of the contribution from each route can be achieved.

### Peak Deconvolution

Several profiles were chosen for peak deconvolution using the procedure described below. The resulting profiles were then analyzed for AUC0-t using Prism software. The ratio of the AUC0-t from each profile against the combined profile is then used to assign a percentage to the transmucosal and oral routes of absorption. The profiles chosen were the average profiles obtained from the four-arm crossover using 15mg DBF fasted upright, 15mg DBF after a moderate fat meal, 15mg DBF after a high fat meal, and lastly, 5mg DBF from the dose proportionality study. The Plasma levels observed for the four average profiles are listed in **Table 9** below.

**Table 9: Diazepam plasma concentrations after administration of DBF under various conditions.**

| Time (hrs) | 0 | 0.25 | 0.5 | 0.75 | 1 | 1.5 | 2 | 3 | 4 | 6 |
|---|---|---|---|---|---|---|---|---|---|---|
| 5mg Fasted | 0.00 | 56.49 | 218.25 | 299.65 | 309.47 | 271.64 | 217.89 | 145.01 | 105.68 | 103.61 |
| 15mg Fasted | 2.27 | 216.03 | 382.65 | 393.67 | 404.78 | 368.19 | 304.46 | 203.59 | 147.63 | 142.53 |
| 15mg Moderat e Fat (MF) | 1.46 | 87.64 | 211.59 | 226.76 | 208.52 | 198.84 | 217.64 | 261.8 | 235.73 | 157.79 |
| 15mg High Fat (HF) | 2.24 | 69.36 | 177.45 | 199.04 | 188.78 | 174.89 | 179.18 | 196.22 | 181.08 | 134.2 |

| Time (hrs) | 9 | 12 | 24 | 48 | 72 | 96 | 120 | 144 | 192 | 240 |
|---|---|---|---|---|---|---|---|---|---|---|
| 5mg Fasted | 103. 47 | 85.64 | 52.36 | 24.45 | 20.23 | 32.48 | 26.05 | 21.50 | 15.69 | 10.17 |
| 15mg Fasted | 128. 82 | 113.64 | 75.72 | 67.65 | 53.99 | 44.02 | 36.25 | 31.41 | 20.7 | 15.82 |
| 15mg Moderat e Fat (MF) | 125. 16 | 116.64 | 80.81 | 65.98 | 53.36 | 41.07 | 36.43 | 28.5 | 19.69 | 14.25 |
| 15mg High Fat (HF) | 118. 62 | 119.15 | 80.09 | 68.17 | 53.08 | 41.3 | 35.35 | 27.5 | 19.05 | 12.7 |

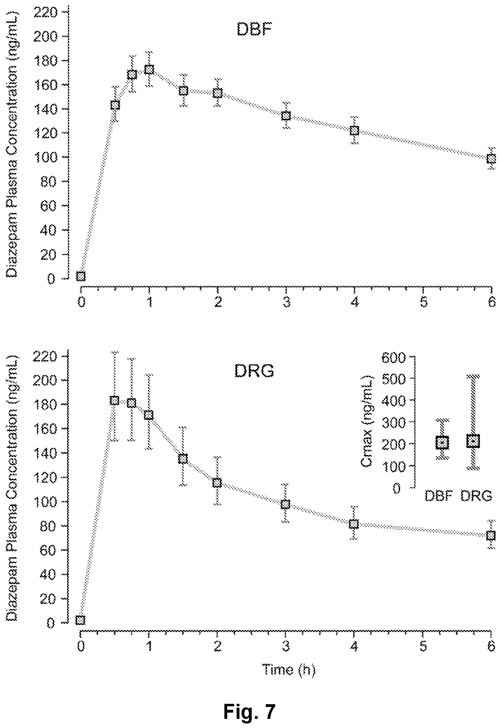

In summary of some of the experiments described herein:
**Rationale:** Diazepam buccal film (DBF) is a novel dosage form of diazepam under development for the management of patients with refractory epilepsy requiring intermittent use of diazepam to control increased seizure activity. We assessed the pharmacokinetic (PK) performance of DBF administered to adults with epilepsy according to a weight-based regimen (dose range 12.5-17.5 mg) compared to diazepam rectal gel (DRG) administered according to the weight-based regimen recommended in the FDA-approved label (dose range 12.5-20 mg).
**Methods:** Adult men and women ages 18-65 years with epilepsy on a stable regimen of ≥1 antiseizure drug (no change in the 30 days prior to receiving study drug and no change anticipated over the course of the study) were enrolled in a 2-period crossover study (NCT03953820) to receive a single dose of either DBF or DRG in randomized sequence and separated by a 28-day washout. Doses were administered within 30 min of a standardized moderate-fat meal. Subjects were confined to the clinic until 24 h after dosing. Diazepam plasma samples were obtained pre-dose and at intervals until 10 d after dosing to enable analysis of maximal plasma concentration (Cₘₐₓ), time to Cₘₐₓ (Tₘₐₓ), area under the curve to the last measurable concentration (AUC_{0-T}), and AUC extrapolated to infinity (AUC_{0-INF}). Subjects were monitored for adverse events (AE) throughout the study.
**Results:** Among 31 subjects enrolled, PK profiles valid for analysis for both DBF and DRG were available for 28 subjects (13 males, 15 females; mean [SD] weight 84.6±20.6 kg). Subjects were excluded from analysis if both treatments were not completed (n=2), or if predose diazepam concentrations were >5% of Cₘₐₓ (n=1). Diazepam mean (SD) dose was 15.4±1.9 mg and 17.1:1:3.0 mg for DBF and DRG, respectively. The **Table 7** shows geometric means for PK parameters with ratio of geometric means (DBF/DRG) for the study population overall (N=28), and geometric means for Cₘₐₓ and corresponding ratios within each weight category. For the study population overall, geometric mean Cₘₐₓ values for DBF and DRG were 204.26 ng/mL (geometric SD [GSD] 136.12-306.49) and 211.22 ng/mL (GSD 87.71-508.63), respectively (see **Figure 7****),** indicating that Cₘₐₓ values following DBF were comparable but significantly less variable than Cₘₐₓ values following DRG (P<0.0001). Values for AUC were higher for DBF than for DRG, and median Tₘₐₓ values for DBF and DRG were 1.0 and 0.52 h, respectively (P<0.05).Three of 28 subjects following DRG dosing failed to achieve a plasma concentration ≥70 ng/mL. There were no serious AEs related to study drug.
**Conclusions:** These results demonstrate that a single dose of DBF administered to adults with epilepsy following a moderate-fat meal according to a weight-based regimen provides exposure to diazepam similar to DRG dosed as recommended with significantly less variability. The geometric mean values for Cₘₐₓ following DBF were consistently ≥150 ng/mL for each of the weight categories.

Another summary follows:

### INTRODUCTION

- Patients with refractory epilepsy may experience seizure exacerbations referred to as "acute repetitive seizures" (ARS), which represent a series of seizures grouped consecutively, typically with short (or shorter than usual) interictal periods.
   - ARS are commonly referred to as "seizure clusters" or "seizure flurries" .
- ARS raise concerns for seizure-associated risks, including postictal psychosis, physical injury, negative social and economic impact from frequent emergency department visits, hospitalizations, or missed school or work days, and for status epilepticus that may lead to persistent neurological impairment or death (Refs. 1-7).
- Despite these increased risks, many patients with epilepsy who experience ARS do not have a rescue medication prescribed and/or a seizure action plan for when cluster seizures occur (Refs. 1, 8-10) .
- Diazepam buccal film (DBF) is a novel dosage form of diazepam under development for the management of patients with refractory epilepsy requiring intermittent use of diazepam to control increased seizure activity (Refs. 11, 12).
   - DBF consists of a rectangular of film smaller than the size of a postage stamp, which is affixed to the buccal mucosa inside the cheek.
   - Diazepam carried within the dissolving polymer matrix of the film is absorbed transmucosally and is also swallowed.
- Currently, diazepam rectal gel (DRG; Diastat^{®} AcuDial^{™} rectal gel, Valeant Pharmaceuticals, Bridgewater, NJ, USA) and midazolam nasal spray (Nayzilam^{®} nasal spray, UCB Biopharma, Smyrna, GA, USA) are the only FDA-approved treatments for breakthrough or cluster seizures (refs. 2, 13-16); these current treatments have certain limitations:
   - Rectal administration can be difficult and time consuming, and is otherwise problematic for many patients and caregivers due to concerns regarding embarrassment and social acceptability (Refs. 13,17,18)
   - Intranasal administration is often poorly accepted by patients, which can negatively impact compliance
- Pharmacokinetic (PK) data from a phase 1 study in healthy adults showed DBF to exhibit greater overall consistency in diazepam exposure (area under the concentration-time curve [AUC], maximal plasma concentration [Cmax]) than DRG (Ref. 12).

### OBJECTIVE

- This study was conducted to compare the PK performance in people with epilepsy of DBF in relation to DRG, the only currently FDA-approved diazepam formulation for treatment of ARS

### STUDY DESIGN AND PATIENTS

• Randomized, multicenter, single-dose, open-label, two-treatment, two-sequence crossover study (NCT03953820).
- Adult patients with epilepsy receiving a stable regimen of antiseizure drugs were randomized to receive a single administration of DBF and a single administration of DRG in crossover fashion.
- There was a 28-day washout phase between doses of study drug.
- DRG was dosed according to the weight-based regimen in the FDA-approved label (dose range 10-20 mg) and DBF was dosed according to a weight-based regimen (dose range 10-17.5 mg) predicted to approximate the PK performance of DRG with respect to Cmax (**Table 10**)
- The treatments were administered following a moderate-fat meal.

**TABLE 10. STUDY DRUG DOSING BY PATIENT WEIGHT CATEGORY**

| Weight | Category (kg) DBF | Dose DRG Dose^{a} |
|---|---|---|
| 38-50 | 10 mg | 10 mg (2 mL) |
| 51-62 | 12.5 mg | 12.5 mg (2.5 mL) |
| 63-75 | 15 mg | 15 mg (3 mL) |
| 76-87 | 15 mg | 17.5 mg (3.5 mL) |
| ≥88 | 17.5 mg | 20 mg (4 mL) |

| | | |
|---|---|---|
| ^{a}DRG was administered according to the weight-based regimen recommended in the FDA-approved label (ref. 16). | | |

• For each dose of study drug, patients were confined to the clinic from approximately 14 hours before dosing until after the 24-hour post-dose blood draw.
- For blood draws after 24 hours post-dose, patients could return to the clinical site or have a sample collected by a home-care nurse.
- While confined in the clinic, patients fasted overnight for at least 10 hours before being served a standardized moderate-fat meal.

### STUDY ASSESSMENTS

- In each period, blood samples for PK analyses were obtained pre-dose and 0.5, 0.75, 1, 1.5, 2, 3, 4, 6, 9, 12, 24, 48, 72, 96, 120, 144, 192, and 240 hours post-dose to enable PK assessments.
   - A window of ±1 minute was allowed for blood samples obtained within the first 8 hours post-dose, ±3 minutes for samples obtained within 8 to 24 hours post-dose, and ±60 minutes for all subsequent blood samples.
- Key PK parameters of interest included Cmax, time to Cmax (Tmax), AUC from time zero to the last non-zero concentration (AUC0-T), and AUC from time zero extrapolated to infinity (AUC0-INF).
- Adverse events (AEs) were monitored throughout the study.

### DATA ANALYSIS

- The safety population included all patients who received at least one dose of study drug; the PK population included all patients who completed period 1 and period 2, had no significant violations of the study protocol, and for whom the PK profile could be adequately characterized.
- PK data were summarized overall and for each weight category using descriptive statistics.
- ANOVA was performed on log-transformed AUC0-T, AUC0-INF, and Cmax at alpha 0.05. Factors in the model were sequence, subject within sequence, period, and treatment. Tmax was analyzed using the non-parametric Wilcoxon signed-rank test.
- The ratios of geometric means with 90% confidence intervals based on least-squares means from ANOVA of log-transformed data were calculated for Cmax, AUC0-T, and AUC0-INF for DBF versus DRG values for all subjects irrespective of weight category, and for subjects within each weight category.

### RESULTS

### PATIENTS

- Among 31 patients enrolled, PK profiles valid for analysis for both DBF and DRG were available for 28 subjects (13 males, 15 females; mean [SD] weight: 84.6 [20.6 kg]).
   - Patients were excluded from PK analyses if both DBF and DRG treatments were not completed (n=2), or if pre-dose diazepam concentrations were >5% of Cmax (n=1).
- Diazepam mean (SD) doses were 15.4 (1.9) mg and 17.1 (3.0) mg for DBF and DRG, respectively.

### PHARMACOKINETICS AND SAFETY

• Geometric means for PK parameters with ratios of the geometric means (DBF/DRG) for the overall study population (N=28 for Cmax and Tmax; N=27 for AUC0-T and AUC0-INF) are shown in **Table 11.**
- AUC0-T and AUC0-INF values were higher for DBF than for DRG
- Overall, Tmax was significantly shorter for DRG versus DBF (P<0.05)
- Three of 28 subjects following DRG dosing failed to achieve a plasma concentration ≥70 ng/mL (70 ng/mL is the estimated threshold plasma concentration of diazepam associated with seizure threshold elevation as determined by a pharmacodynamic-PK study in rats) (ref. 19).

**TABLE 11. PHARMACOKINETIC PARAMETERS FOLLOWING DBF AND DRG ADMINISTERED TO ADULTS WITH EPILEPSY FOLLOWING A MODERATE-FAT MEAL IN THE OVERALL STUDY POPULATION (N=28)**

| Parameter | DBF | DRG | Ratio of Geometric Means, DBF/DBG (%)^{a} | 90% CI (%) for Ratio^{b} |
|---|---|---|---|---|
| Cmax (ng/mL), geometric mean | 204.26 | 211.22 | 96.70 | 70.53, 132.58 |
| AUC(0-T) (ng•h/mL), Geometric mean^{c} | 7290.40 | 5682.09 | 128.31 | 95.93, 171.61 |
| AUC(0-INF) (ng•h/mL), Geometric mean^{c} | 8672.09 | 6880.96 | 126.03 | 103.67, 153.21 |
| Tmax (h), median | 1.0 | 0.517^{d} NA | | |

| | | | | |
|---|---|---|---|---|
| ^{a}Calculated using least-square means according to the formula e(Difference) x 100. ^{b}90% geometric confidence interval using log-transformed data. ^{c}N=27. ^{d}P<0.05 vs DBF. | | | | |

• Geometric mean diazepam plasma concentrations over time in the overall study population following DBF and DRG are illustrated in **Figure 7****.**
- For the study population overall, geometric mean Cmax values following DBF and DRG administration were 204.26 ng/mL (geometric SD [GSD]: 136.12-306.49) and 211.22 ng/mL (GSD 87.71-508.63), respectively, indicating that Cmax values following DBF were comparable but significantly less variable than Cmax values following DRG (*P*<0.0001) (**Table 11** and **Figure 7** **inset**).

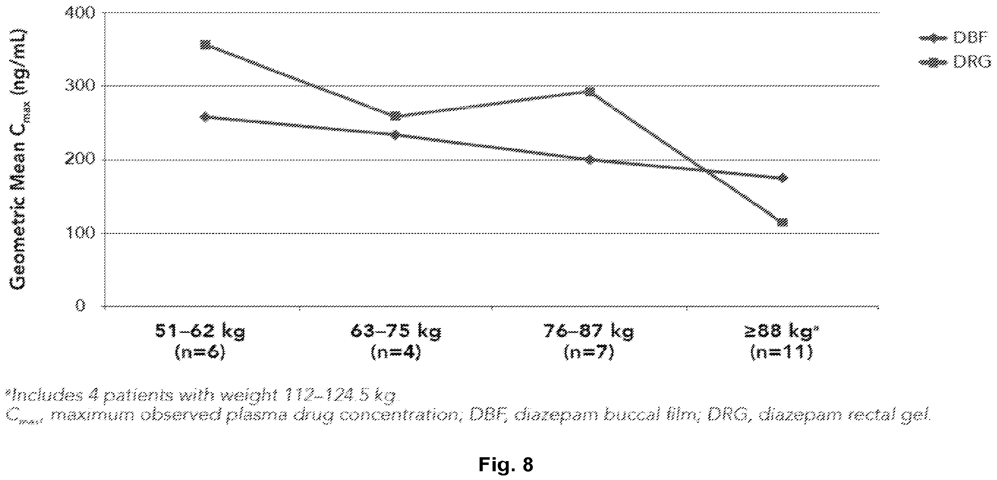

Values graphed are geometric mean (geometric SE) plasma concentrations. Inset shows geometric mean (geometric SD) Cmax values. Cmax, maximum observed plasma drug concentration; DBF, diazepam buccal film; DRG, diazepam rectal gel; SD, standard deviation; SE, standard error.
• Geometric means for Cmax and corresponding ratios within each weight category are shown in **Table 12.**
- Cmax values were less variable with DBF versus DRG (Figure 8).

**TABLE 12. DBF AND DRG MAXIMAL PLASMA CONCENTRATIONS (CMAX) ACCORDING TO WEIGHT CATEGORY (N=28)**

| Cmax Geometric | DBF | DRG | Ratio of Cmax | 90% CI (%) |
|---|---|---|---|---|
| Mean by Weight Group | | | Geometric Means DBF/DRG (%)^{a} | for Ratio^{b} |
| 51-62 kg (n=6) | | | | |
| Cmax (ng/mL) | 258.38 | 358.06 | 72.16 | 51.17, 101.76 |
| Dose (mg) | 15.0 | 15.0 | - | - |
| 76-87 kg (n=7) | | | | |
| Cmax (ng/mL) | 201.39 | 293.00 | 68.74 | 46.77, 101.01 |
| Dose (mg) | 15.0 | 17.5 | - | - |
| ≥88c (n=11) | | | | |
| Cmax (ng/mL) | 175.56 | 115.82 | 151.58 | 71.59, 320.94 |
| Dose (mg) | 17.5 | 20.0 | - | - |

| | | | | |
|---|---|---|---|---|
| ^{a}Calculated using least-square means according to the formula e(Difference) x 100. ^{b}90% geometric confidence interval using log-transformed data. ^{c}The highest weight category included 4 individuals with body weight 112-124.5 kg. CI, confidence interval; Cmax, maximum observed plasma drug concentration; DBF, diazepam buccal film; DRG, diazepam rectal gel. | | | | |

• There were no serious AEs related to study drug reported.

### CONCLUSIONS

- These results demonstrate that a single dose of DBF provides similar exposure to diazepam as DRG with significantly less variability when administered to adults with epilepsy according to weight-based regimens following a moderate-fat meal.
- The geometric mean values for Cmax following DBF were consistently ≥150 ng/mL for each of the weight categories.
- These results support further development of DBF as an easily administered alternative to DRG for patients with epilepsy who experience breakthrough or cluster seizures despite treatment with antiseizure medications.

### REFERENCES.

**1.** Penovich PE, et al. Neurologist. 2017;22:207-14.
**2.** Haut SR. Curr Opin Neurol. 2015;28:143-50.
**3.** Kanner AM, et al. Arch Neurol. 1996;53:258-63.
**4.** Pellock JM. J Child Neurol. 2007;22:9S-13S.
**5.** Sillanpaa M, Schmidt D. Brain. 2008;131:938-44*.*
**6.** Bergen DC. Epilepsy Curr. 2006;6:117-8.
**7.** Haut SR, et al. Epilepsia. 1999;40:1832-4.
**8.** Detyniecki K, et al. Epilepsy Behav. 2018;88:349-56.
**9.** Gainza-Lein M, et al. Seizure. 2017;52:188-94.
**10.** Vigevano F, et al. Eur J Paediatr Neurol. 2018;22:56-63.
**11.** Heller AH, et al. Neurology. 2018;90(15 suppl):P4.272.
**12.** Heller AH, et al. Neurology. 2018;90(15 suppl):P4.273.
**13.** Cereghino JJ. Curr Treat Options Neurol. 2007;9:249-55.
**14.** Jafarpour S, et al. Seizure. 2019;68:9-15.
**15.** Nayzilam [package insert]. Smyrna, GA: UCB, Inc.; 2019.
**16.** Diastat C-IV (diazepam rectal gel) [package insert]. San Antonio, TX: DPT Laboratories; 2016.
**17.** Fisgin T, et al. J Child Neurol. 2002;17:123-6.
**18.** Tatum IW. Epilepsy Behav. 2002;3:535-8.
**19.** Dhir A, Rogawski MA. Epilepsia. 2018;59:935-44.

## Claims

1. Diazepam for use in the treatment of seizures, wherein the diazepam is orally administered with a multimodal delivery profile,
wherein the diazepam is delivered from a mucoadhesive matrix including a permeation enhancer,
wherein permeation of at least a portion of the diazepam through a mucosal tissue is promoted, and wherein a dose of diazepam is adjusted to compensate for a food effect by increasing the dose to offset the food effect such that a subject achieves a safe and efficacious dose in a fed state.

2. Diazepam for the use of claim 1, wherein the multimodal profile is a bimodal delivery profile.

3. Diazepam for the use of claim 1, wherein at least about 5-50 % of the diazepam is administered via an oral transmucosal route.

4. Diazepam for the use of claim 1, wherein at least about 5% of the diazepam is administered via a gastrointestinal route.

5. Diazepam for the use of claim 1, wherein the diazepam is for use in the treatment of generalized seizures, focal seizures, focal aware seizures, focal aware impaired seizures, bilateral tonic seizures, absence seizures, atypical absence seizures, tonic-clonic seizures, atonic seizures, or clonic seizures.

6. Diazepam for the use of claim 1, wherein the diazepam is for use in the treatment of tonic seizures, myoclonic seizures, gelastic and dacrystic seizures, febrile seizures, non-epileptic seizures or refractory seizures.

7. Diazepam for the use of claim 1, wherein the diazepam is administered in a fed state or in a fasted state.

8. Diazepam for the use of claim 1, wherein about 2.5 mg, about 5mg, about 7.5 mg, about 10 mg, about 12.5 mg, about 15 mg, about 17.5 mg, about 20 mg, about 25 mg, or about 30 mg of diazepam is delivered in a single dose.

9. Diazepam for the use of claim 1, wherein a dose of diazepam is administered according to a weight-based regimen.

10. Diazepam for the use of claim 1, wherein the matrix is a pharmaceutical film with a residence time of less than 30 minutes, less than 15 minutes, less than 10 minutes or less than 5 minutes in an oral cavity.

## Patentansprüche

1. Diazepam zur Verwendung bei der Behandlung von Krampfanfällen, wobei das Diazepam oral mit einem multimodalen Verabreichungsprofil verabreicht wird,
wobei das Diazepam aus einer mukoadhäsiven Matrix, die einen Permeationsverstärker enthält, zugeführt wird,
wobei die Permeation wenigstens eines Teils des Diazepams durch ein Schleimhautgewebe gefördert wird und wobei eine Diazepamdosis zur Kompensierung eines Nahrungsmitteleffekts eingestellt wird, indem die Dosis zum Ausgleichen des Nahrungsmitteleffekts so erhöht wird, dass ein Individuum in einem genährten Zustand eine sichere und wirksame Dosis erreicht.

2. Diazepam zur Verwendung nach Anspruch 1, wobei es sich bei dem multimodalen Profil um ein bimodales Zuführungsprofil handelt.

3. Diazepam zur Verwendung nach Anspruch 1, wobei mindestens etwa 5-50 % des Diazepams über einen oralen transmukosalen Weg verabreicht werden.

4. Diazepam zur Verwendung nach Anspruch 1, wobei mindestens etwa 5 % des Diazepams über einen Magen-DarmTrakt-Weg verabreicht werden.

5. Diazepam zur Verwendung nach Anspruch 1, wobei das Diazepam zur Verwendung bei generalisierten Anfällen, fokalen Anfällen, fokalen Anfällen ohne Bewusstseinsstörung, fokalen Anfällen mit Bewusstseinsstörung, bilateralen tonischen Anfällen, Absence-Anfällen, atypischen Absence-Anfällen, tonischklonischen Anfällen, atonischen Anfällen oder klonischen Anfällen vorgesehen ist.

6. Diazepam zur Verwendung nach Anspruch 1, wobei das Diazepam zur Verwendung bei der Behandlung von tonischen Anfällen, myoklonischen Anfällen, gelastischen und dakrystischen Anfällen, Fieberkrämpfen, nicht epileptischen Anfällen oder refraktären Anfällen vorgesehen ist.

7. Diazepam zur Verwendung nach Anspruch 1, wobei das Diazepam in einem genährten Zustand oder in einem nüchternen Zustand verabreicht wird.

8. Diazepam zur Verwendung nach Anspruch 1, wobei etwa 2,5 mg, etwa 5 mg, etwa 7,5 mg, etwa 10 mg, etwa 12,5 mg, etwa 15 mg, etwa 17,5 mg, etwa 20 mg, etwa 25 mg oder etwa 30 mg Diazepam in einer Einzeldosis zugeführt werden.

9. Diazepam zur Verwendung nach Anspruch 1, wobei eine Diazepamdosis gemäß einem gewichtsbezogenen Schema verabreicht wird.

10. Diazepam zur Verwendung nach Anspruch 1, wobei es sich bei der Matrix um einen pharmazeutischen Film mit einer Verweilzeit von weniger als 30 Minuten, weniger als 15 Minuten, weniger als 10 Minuten oder weniger als 5 Minuten in einer Mundhöhle handelt.

## Revendications

1. Diazépam pour une utilisation dans le traitement de crises convulsives, dans lequel le diazépam est administré oralement avec un profil d'administration multimodal,
dans lequel le diazépam est administré à partir d'une matrice mucoadhésive comprenant un activateur de perméation,
dans lequel la perméation d'au moins une partie du diazépam à travers un tissu muqueux est favorisée, et dans lequel une dose de diazépam est ajustée pour compenser un effet alimentaire en augmentant la dose pour compenser l'effet alimentaire de sorte qu'un sujet atteint une dose sûre et efficace dans un état nourri.

2. Diazépam pour l'utilisation selon la revendication 1, dans lequel le profil multimodal est un profil d'administration bimodal.

3. Diazépam pour l'utilisation selon la revendication 1, dans lequel au moins environ 5-50 % du diazépam est administré par une voie transmuqueuse orale.

4. Diazépam pour l'utilisation selon la revendication 1, dans lequel au moins environ 5 % du diazépam est administré par une voie gastro-intestinale.

5. Diazépam pour l'utilisation selon la revendication 1, dans lequel le diazépam est pour une utilisation dans le traitement des crises généralisées, des crises focales, des crises focales conscientes, des crises focales conscientes altérées, des crises toniques bilatérales, des crises d'absence, des crises d'absence atypiques, des crises tonico-cloniques, des crises atoniques ou des crises cloniques.

6. Diazépam pour l'utilisation selon la revendication 1, dans lequel le diazépam est pour une utilisation dans le traitement de crises toniques, de crises myocloniques, de crises gélastiques et dacrystiques, de crises fébriles, de crises non épileptiques ou de crises réfractaires.

7. Diazépam pour l'utilisation selon la revendication 1, dans lequel le diazépam est administré dans un état nourri ou dans un état à jeun.

8. Diazépam pour l'utilisation selon la revendication 1, dans lequel environ 2,5 mg, environ 5 mg, environ 7,5 mg, environ 10 mg, environ 12,5 mg, environ 15 mg, environ 17,5 mg, environ 20 mg, environ 25 mg ou environ 30 mg de diazépam sont administrés en une seule dose.

9. Diazépam pour l'utilisation selon la revendication 1, dans lequel une dose de diazépam est administrée selon un régime basé sur le poids.

10. Diazépam pour l'utilisation selon la revendication 1, dans lequel la matrice est un film pharmaceutique ayant un temps de séjour inférieur à 30 minutes, inférieur à 15 minutes, inférieur à 10 minutes ou inférieur à 5 minutes dans une cavité buccale.
